(19) Europäisches Patentamt
European Patent Office
Office européen des brevets

(11) EP 3 108 252 B1

(12) FASCICULE DE BREVET EUROPEEN

(45) Date de publication et mention de la délivrance du brevet:
09.10.2019 Bulletin 2019/41

(51) Int Cl.:
C12Q 1/6883 (2018.01)  G01N 33/68 (2006.01)
G01N 33/50 (2006.01)

(21) Numéro de dépôt: 15709253.7

(22) Date de dépôt: 16.02.2015

(86) Numéro de dépôt international:
PCT/FR2015/050365

(87) Numéro de publication internationale:
WO 2015/121596 (20.08.2015 Gazette 2015/33)

(54) **METHODE PREDICTIVE POUR CARACTERISER LA RADIOSENSIBILITE ET LA REACTION TISSULAIRE D'UN PATIENT ENVERS UN RAYONNEMENT IONISANT THERAPEUTIQUE**

PRÄDIKTIVES VERFAHREN ZUR CHARAKTERISIERUNG DER RÖNTGENSTRAHLENEMPFINDLICHKEIT UND GEWEBEREAKTION EINES PATIENTEN AUF THERAPEUTISCHE IONISIERENDE STRAHLUNG

PREDICTIVE METHOD OF CHARACTERIZING THE RADIOSENSITIVITY AND TISSULAR REACTION OF A PATIENT TO THERAPEUTIC IONIZING RADIATION

(84) Etats contractants désignés:
AL AT BE BG CH CY CZ DE DK EE ES FI FR GB GR HR HU IE IS IT LI LT LU LV MC MK MT NL NO PL PT RO RS SE SI SK SM TR

(30) Priorité: 17.02.2014 FR 1451215
17.02.2014 FR 1451216
10.10.2014 FR 1402282
10.10.2014 FR 1402281

(43) Date de publication de la demande:
28.12.2016 Bulletin 2016/52

(73) Titulaires:
• Université Claude Bernard Lyon 1
69622 Villeurbanne, Cedex (FR)
• Centre National de la Recherche Scientifique
75794 Paris Cedex 16 (FR)
• Centre Léon Bérard
69008 Lyon (FR)
• Institut National de la Santé et de la Recherche Médicale
75654 Paris 13 (FR)

(72) Inventeurs:
• FORAY, Nicolas
38300 Meyrie (FR)
• GRANZOTTO, Adeline
69008 Lyon (FR)
• DEVIC, Clément
69003 Lyon (FR)

(74) Mandataire: Schmidt, Martin Peter
IXAS Conseil
15, rue Emile Zola
69002 Lyon (FR)

(56) Documents cités:
• Nicolas Foray: "Les réparatoses : nouveaux concepts sur la prédiction de la radiosensibilité", , 25 janvier 2008 (2008-01-25), XP055131242, Extrait de l'Internet: URL:http://www-sante.ujf-grenoble.fr/SANTE/alpesmed/evenements/rns/Rencontres_nuclaires_annees_precedentes/rencontresnucleaires2008/pdf/25_01_08/04_Foray.pdf [extrait le 2014-07-23]
• A Granzotto ET AL: "Towards a first classification of human radiosensitivity: radiotherapy, radiodiagnosis and history of radiobiology HIGH DOSES STUDIES - THE HUMAN RADIOSENSITIVITY", , 30 mars 2012 (2012-03-30), XP055131016, Extrait de l'Internet: URL:http://www.crcl.fr/Portals/0/Equipes/Equipe AnsieauPuisieux/poster radiobiology group (2).pdf [extrait le 2014-07-23]

- JOUBERT A ET AL: "Irradiation in presence of iodinated contrast agent results in radiosensitization of endothelial cells: Consequences for computed tomography therapy", INTERNATIONAL JOURNAL OF RADIATION: ONCOLOGY BIOLOGY PHYSICS, PERGAMON PRESS, USA, vol. 62, no. 5, 1 août 2005 (2005-08-01), pages 1486-1496, XP027750052, ISSN: 0360-3016 [extrait le 2005-08-01]
- JOUBERT ET AL: "Radiosensibilite intrinseque et cassures double-brin de l'ADN dans les cellules humaines", CANCER RADIOTHERAPIE, ELSEVIER, PARIS, FR, vol. 11, no. 3, 23 février 2007 (2007-02-23), pages 129-142, XP022089743, ISSN: 1278-3218, DOI: 10.1016/J.CANRAD.2007.01.003
- CHARLES THOMAS ET AL: "Low-dose hyper-radiosensitivity of progressive and regressive cells isolated from a rat colon tumour: Impact of DNA repair", INTERNATIONAL JOURNAL OF RADIATION BIOLOGY, vol. 84, no. 7, 1 juillet 2008 (2008-07-01), pages 533-548, XP055130893, ISSN: 0955-3002, DOI: 10.1080/09553000802195331
- CHARLES THOMAS ET AL: "Impact of dose-rate on the low-dose hyper-radiosensitivity and induced radioresistance (HRS/IRR) response*", INTERNATIONAL JOURNAL OF RADIATION BIOLOGY, vol. 89, no. 10, 1 octobre 2013 (2013-10-01), pages 813-822, XP055130896, ISSN: 0955-3002, DOI: 10.3109/09553002.2013.800248
- Michel Bourguignon: "Actualités en radioprotection Seuil probabiliste chez l'adulte", , 10 février 2012 (2012-02-10), XP055131240, Extrait de l'Internet: URL:http://www-sante.ujf-grenoble.fr/SANTE /alpesmed/evenements/rns/Rencontres_nuclai res_annees_precedentes/rencontresnucleaire s_sante2012/pdf/vendredi/m_bourguignon_ven dredi_10_fevrier_RNS_2012.pdf [extrait le 2014-07-23]
- COLIN C ET AL: "MRE11 and H2AX biomarkers in the response to low-dose exposure: balance between individual susceptibility to radiosensitivity and to genomic instability", INTERNATIONAL JOURNAL OF LOW RADIATION, INDERSCIENCE PUBLISHERS, vol. 8, no. 2, 13 décembre 2011 (2011-12-13), pages 96-106, XP009179289, ISSN: 1477-6545, DOI: 10.1504/IJLR.2011.044191
- Michel Bolla: "Echelle ASN-SFRO pour la prise en compte des événements de radioprotection affectant des patients dans le cadre d'une procédure médicale de Radiothérapie", , 18 février 2009 (2009-02-18), XP055180247, Extrait de l'Internet: URL:http://www-sante.ujf-grenoble.fr/SANTE /alpesmed/evenements/rns/Rencontres_nuclai res_annees_precedentes/rencontresnucleaire s2009/pdf/02_M_Bollat.pdf [extrait le 2015-03-30]

**Description**

**Domaine technique de l'invention**

L'invention concerne le domaine de la radiobiologie médicale, et plus particulièrement le domaine des méthodes de laboratoire radiobiologiques. L'invention concerne une nouvelle méthode prédictive de la radiosensibilité cellulaire, tissulaire et clinique, qui se base sur la détermination et le recoupement de plusieurs paramètres et critères cellulaires et enzymatiques.

**Etat de la technique**

Environ 1 à 15% des patients traités par radiothérapie pour un cancer montrent une réaction tissulaire (tel qu'une dermite ou une rectite) qui peut mettre en jeu le bon déroulement du traitement dans la mesure où elle peut conduire le médecin à décider l'arrêt du traitement radiothérapeutique avant la fin du protocole prévu. Par ailleurs, cette réaction tissulaire est un indicateur d'une sensibilité particulièrement élevée du patient au rayonnement ionisant. Ainsi, le traitement radiothérapeutique, même interrompu au moment de l'apparition des premiers signes tissulaires visibles, peut augmenter la morbidité voire la mortalité post-traitement des patients, non seulement parce que le cancer qu'il était censé traiter n'a pas pu être éradiqué complètement dû à l'arrêt prématuré du traitement, mais encore à cause de l'endommagement collatéral des tissus sains induit par le rayonnement lui-même.

On sait également que la question de la sensibilité des tissus au rayonnement ionisant est inséparable de celles des mécanismes de réparation des dommages de l'ADN. En effet, au niveau cellulaire, le rayonnement ionisant peut casser certains types de liaisons chimiques en générant des radicaux libres (en particulier par peroxydation) et d'autres espèces réactives à l'origine des dommages de l'ADN. L'endommagement de l'ADN par des agressions endogènes ou exogènes (telles que les radiations ionisantes et les radicaux libres), peut aboutir à différents types de dommages de l'ADN en fonction notamment de l'énergie déposée : les dommages de bases, les cassures simple-brin et les cassures double-brin (CDB). Les CDB non réparées sont associées à la mort cellulaire, la toxicité et plus spécifiquement la radiosensibilité. Les CDB mal réparées sont associées à l'instabilité génomique, aux phénomènes mutagènes et à la prédisposition au cancer. L'organisme dispose de systèmes de réparation spécifiques à chaque type de dommage de l'ADN. En ce qui concerne les CDB, les mammifères possèdent deux principaux modes de réparation : la réparation par suture (ligation des brins) et la réparation par recombinaison (insertion d'un brin homologue ou non-homologue).

On sait que la sensibilité des tissus au rayonnement ionisant est très variable d'un organe à l'autre et d'un individu à l'autre ; l'idée d'une « radiosensibilité intrinsèque » a été conceptualisée par Fertil et Malaise en 1981. Ainsi, les diverses études sur les effets thérapeutiques et les effets secondaires de la radiothérapie ont montré qu'il existe des individus qui jouissent d'une radiorésistance particulièrement élevée, et des individus qui montrent au contraire une radiosensibilité qui peut aller d'un effet secondaire reconnu cliniquement mais sans conséquence jusqu'à un effet létal. Même en dehors de certains rares cas de radiosensibilité extrême, dont l'origine génétique semble avérée, on pense que la radiosensibilité découle d'une manière générale d'une prédisposition génétique : elle est donc propre à un individu. Il serait donc désirable de disposer d'une méthode d'essai prédictive pour pouvoir déterminer la dose maximale cumulée qu'un patient donné peut recevoir sans risque. Cette question se pose en premier lieu en radiothérapie dans un contexte de fortes doses ionisantes. Toutefois, cette question est également susceptible de se poser pour toute autre exposition à de fortes doses ionisantes, équivalentes à celles utilisées en radiothérapie.

On sait que deux protéines de la famille des kinases, appelées couramment ATM et ATR, sont impliquées dans la détection, la réparation et la signalisation des CDB ; leur action nécessite au moins la présence d'une protéine connue sous la désignation BRCA1 et d'une cascade de phosphorylations ordonnée des différents substrats d'ATM (voir l'article de N. Foray et al., « A subset of ATM- and ATR-dependent phosphorylation events requires the BRCA1 protein », paru dans The EMBO Journal vol. 22(11), p. 2860-2871 (2003)). L'essai a été entrepris d'utiliser l'enzyme ATM dans un modèle explicatif de la radiosensibilité cellulaire (voir Joubert et al., "DNA double-strand break repair defects in syndromes associated with acute radination response ; At least two different assays to predict intrinsic radiosensitivity ?", paru dans Int. J. Radiat. Biol., vol. 84(2), p. 107-125 (2008)), et cela a permis d'identifier trois types de radiosensibilité : les cellules radiorésistantes (radiosensibilité dite de Groupe I), les cellules modérément radiosensibles (radiosensibilité dite de Groupe II), et les cellules extrêmement radiosensibles (radiosensibilité dite de Groupe III). Cependant, aucun modèle prédictif n'a été proposé sur cette base, notamment aucune relation quantitative n'a été établie entre les données cliniques (grade de sévérité tissulaire de 0 à 5) et les données radiobiologiques. De même, la présentation de N. Foray, « Les réparatoses : nouveaux concepts sur la prédiction de la radiosensibilité », effectuée lors des « Rencontres Nucléaire & Santé » du 25 janvier 2008 (XP55131242) évoque le rôle des différents marqueurs pH2AX et MRE11 et leurs évolutions au cours du temps pour décrire le nombre de cassures double-brin radioinduites. Cette présentation ne fait pas non plus mention des grades de sévérité tissulaires qui quantifient et répertorient le niveau de radiosensibilité observé au niveau clinique.

De nombreux documents décrivent les conditions dans lesquelles l'ATM peut contribuer à la détection et la réparation de l'endommagement de l'ADN. La demande de brevet WO 2004/013634 (KUDOS Pharmaceuticals Ltd) décrit l'identification d'une composante de voie de signalisation de dommage ATM-dépendant de l'ADN qui interagit avec d'autres facteurs de réponse aux dommages de l'ADN, y compris le complexe MRE11/Rad51/NBS1. La demande de brevet US 2007/0072210 (Ouchi et Aglipay) propose une méthode de criblage d'agents thérapeutiques potentiels qui favorisent une réponse aux dommages de l'ADN, dans laquelle on mélange une protéine appelée BAAT1 (qui est associée à une prédisposition au cancer du sein liée au gène BRCA1), une protéine ATM et le composé candidat ; si la phosphorylation de l'ATM est augmentée par rapport à un mélange témoin ne comportant pas le composé candidat, ce dernier est identifié comme un potentiel principe actif favorisant la réparation de l'ADN. La demande de brevet EP 2 466 310 A1 (Helmholtz Zentrum München) décrit la réparation de ruptures de l'ADN de type double brin en présence de la forme phosphorylée de l'histone H2AX (appelée gamma-H2AX ou g-H2AX). La demande WO 00/47760 et le brevet US 7,279,290 (St. Jude's Children's Research Hospital) décrivent le rôle de la fonction kinase de l'ATM dans la réparation de l'ADN.

Ces derniers documents décrivent donc des voies de réparation mais n'offrent aucune corrélation pour établir un lien avec la clinique.

Le brevet EP 1 616 011 B1 (International Centre for Genetic Engineering and Biotechnology) propose une méthode pour le diagnostic d'un défaut génétique dans la réparation de l'ADN basée sur trois étapes : la culture de cellules isolées d'un échantillon à tester, l'incubation de ces cellules avec un polypeptide chimérique, la caractérisation de la réponse cellulaire. Ladite réponse cellulaire est le taux d'expression d'un marqueur biochimique consistant en protéines intracellulaires de type p53, ATM, Chk1, Chk2, BRCA1, BRCA2, Nbs1, MRE11, Rad50, Rad51 et des histones. Toutefois, l'expression radioinduite peut ne pas être prédictive de la fonctionnalité de ces protéines (certains syndromes présentent un taux d'expression normale alors que la protéine est mutée) : ces procédures ne sont pas des tests fonctionnels.

Les demandes de brevet WO 01/90408, WO 2004/059004 et WO 2006/136686 (Commissariat à l'Energie Atomique) décrivent d'autres méthodes pour constater l'endommagement de l'ADN suite à une irradiation ionisante. Le premier document concerne la mise en évidence d'activités d'incision des lésions de l'ADN, il ne permet pas la quantification des activités enzymatiques d'excision et de resynthèse de l'ADN, ni la réparation des CDB. Les deux autres documents décrivent l'évaluation quantitative des capacités d'un milieu biologique à réparer son ADN à l'aide d'ADN double-brin circulaire superenroulé (selon le troisième document : immobilisé dans un film d'hydrogel de polyacrylamide poreux). Ces procédés ne concernent pas directement les CDB dans leur environnement physiologique *in situ* et aucune corrélation n'existe pour valider leur application en clinique.

KR20030033519 propose de déduire la sensibilité au rayonnement de l'activité de la catalyse ou de la superoxyde dismutase, KR20030033518 utilise la glutathione peroxydase ou la glucose 6-phosphate dehydrogenase. De telles méthodes ne détectent pas des marqueurs directement reliés à l'endommagement ou à la réparation de l'ADN.

La demande de brevet US 2011/312514 (Dana Farber Cancer Institute) propose d'utiliser la détection des foci FANCD2 comme marqueur. La demande de brevet US 2007/0264648 (National Institute of Radiological Sciences) propose l'utilisation des oligomères de l'ADN pour la prédiction de l'apparition d'effets secondaires lors des radiothérapies. Toutefois, certaines radiosensibilités peuvent être observées alors que le taux de foci FANCD2 est normal.

Les demandes de brevet US 2008/234946 et US 2012/041908 (University of South Florida et al.) décrivent une méthode pour la prédiction de la radiosensibilité de cellules cancéreuses, et non pas de cellules saines ; elle est en plus basée sur des données génomiques et non des tests fonctionnels.

La demande de brevet WO2014/154854 (Centre Hospitalier Universitaire de Montpellier) décrit une méthode pour la prédiction de la radiosensibilité d'un sujet via l'utilisation d'au moins un biomarqueur de radiosensibilité. Cette méthode ne détecte pas des marqueurs directement reliés à l'endommagement ou à la réparation de l'ADN ; elle est en plus basée sur des données protéomiques. De plus, cette demande de brevet ne décrit pas de relation quantitative entre les données radiobiologiques et la sévérité des réactions tissulaires.

La demande de brevet WO 2013/187973 (University of California) décrit des systèmes et des méthodes de détermination de la radiosensibilité de cellules et/ou d'un sujet au regard d'une population contrôle. Plus particulièrement cette méthode inclut l'irradiation d'un échantillon biologique, la détection et la quantification de foci radioinduits au sein de cellules erythrocytes, lymphocytes ou de cellules primaires, résultant d'un prélèvement sanguin via l'utilisation d'un ou plusieurs marqueurs de détection parmi un ensemble de marqueurs dont l'anti-pH2AX, l'anti-MRE11 et l'anti-ATM. La quantification des foci radioinduits à différents temps d'observation post-irradiation inférieurs à 2 h permet de déterminer leur cinétique de réparation qui est corrélée de manière empirique à la radiosensibilité du sujet. Toutefois, l'analyse des focis dans des cellules de type lymphocyte est très difficile du fait de leur petit noyau. De plus, cette méthode ne permet cependant pas au praticien de prendre des décisions quant au traitement du patient.

Le brevet US 8,269,163 (New York University School of Medecine) décrit un grand nombre de protéines susceptibles d'être utilisées comme marqueurs pour apprécier de manière simple et rapide l'importance de l'exposition accidentelle au rayonnement ionisant qu'une personne a subie, en vue de trier les patients et de les orienter vers un traitement d'urgence approprié. Ce dernier brevet concerne la dosimétrie biologique (détermination de la dose accidentelle) alors que la détection de la radiosensibilité s'effectue à partir d'une dose connue.

La demande de brevet WO 2010/88650 (University of Texas) décrit des méthodes et compositions pour identifier les cellules cancéreuses qui sont soit sensibles soit résistantes à un traitement de radiothérapie particulier ; elles n'est donc pas applicable à tout traitement radiothérapeutique.

La demande de brevet WO 2010/136942 (Philips) décrit une méthode globale pour surveiller un patient lors d'une radiothérapie à l'aide de biomarqueurs. Le procédé comprend l'obtention d'au moins un descripteur dérivé d'une image extrait d'une modalité d'image, dans lequel le descripteur appartient à un tissu d'intérêt pour lequel est prévue une radiothérapie ou à un tissu dans le voisinage de ce volume cible. Le procédé comprend en outre la sélection d'au moins un marqueur biologique spécifique d'une maladie apte à détecter ou quantifier des effets secondaires de la radiothérapie dans la zone des tissus d'intérêt. En outre, le procédé comprend la récupération d'au moins une valeur de mesure in vitro du biomarqueur spécifique de la maladie sélectionnée. En outre, le procédé comprend le traitement du au moins descripteur de la au moins une valeur de biomarqueur in vitro au moyen d'une technique de corrélation, résultant en un signal de sortie indicatif de la radiotoxicité dans la région du tissu d'intérêt. Toutefois, l'enseignement de ce brevet ne tient compte que de la toxicité dépendant du tissu et non de l'individu et est principalement basée sur l'analyse d'images.

La demande de brevet WO 2010/109357 décrit un procédé et un appareil pour la planification de protocole de radiothérapie adaptative basée sur l'optimisation de la probabilité de complication des tissus normaux et la probabilité de contrôle tumoral selon des marqueurs spécifiques à chaque patient. Les valeurs des marqueurs associées à des tissus normaux comprennent des valeurs de tests in vitro, les signatures par spectrométrie de masse des protéines, des données de l'anamnèse et les antécédents du patient. Les valeurs d'essai in vitro peuvent être d'origine cellulaire, protéomique et génétique tel que, mais sans s'y limiter, divers comptages de cellules, HB, CRP, PSA, TNF-alpha, ferritine, transferrine, LDH, IL-6, l'hepcidine, créatinine, glucose, HbAlc, et la longueur des télomères. Les marqueurs de l'anamnèse et des antécédents du patient incluent la chirurgie abdominale antérieure, les médicaments hormonaux ou de anticoagulants, le diabète, l'âge, et les mesures liées à la croissance tumorale. Les biomarqueurs non liés à la radiotoxicité sont également envisagés, tels que les biomarqueurs associés à diverses formes d'ablation ou agents chimiothérapeutiques. Toutefois, la radiosensibilité individuelle n'y est pas prise en compte.

Malgré ce vaste état de la technique, la demanderesse constate que les brevets décrits ci-dessus ne décrivent pas une méthode de quantification de la radiosensibilité individuelle permettant d'évaluer le risque de réactions tissulaires post-radiothérapie, qui puisse être employé pour tout patient et tout type de rayonnement ionisant susceptible d'induire des CDB, et qui soit prédictif. Le problème de founir une méthode prédictive de la radiosensibilité individuelle reste donc sans solution opérationnelle. La présente invention vise à proposer une nouvelle méthode prédictive de la radiosensibilité tissulaire et clinique.

**Objets de l'invention**

Les inventeurs ont constaté, et la méthode selon l'invention part de ce constat, que les cassures double-brin (CDB) de l'ADN sont les dommages radioinduits les plus prédictifs de la radiosensibilité quand ils sont non-réparés d'une part, et de l'instabilité génomique quand ils sont mal réparés d'autre part. Les inventeurs ont découvert que les CDB sont prises en charge par le mode majoritaire de réparation dit de suture, et/ou par le mode minoritaire de réparation fautive dit recombinaison MRE11-dépendante. L'équilibre entre ces deux modes de réparation est contrôlé par la protéine ATM. Le marqueur pH2AX indique un site CDB reconnu par le mode de réparation par suture. Le marqueur MRE11 indique un site de CDB qui a été pris en charge par la réparation fautive MRE11-dépendante. Le marqueur pATM renseigne sur l'activation de la voie de suture par phosphorylation de H2AX et l'inhibition de la voie MRE11-dépendante.

Les inventeurs ont par ailleurs observé un transfert des formes cytoplasmiques de la protéine ATM dans le noyau cellulaire à la suite d'un stress de type oxydatif, et notamment à la suite d'un stress lié à un rayonnement ionisant induisant des CDB.

Pour constater l'endommagement d'un ADN par une aggression exogène, il faut tenir compte, d'une part, de l'état spontané de l'ADN, et, d'autre part, de son état radioinduit. Par ailleurs, après irradiation, il faut tenir compte de la réparation de l'ADN, dont la cinétique dépend du mécanisme de réparation et donc du type d'endommagement radioinduit. On sait par ailleurs que l'efficacité et la rapidité de la réparation de l'ADN varie d'un individu à l'autre, et qu'il existe en outre des conditions génétiques particulières qui conduisent à une radiosensibilité exceptionnelle.

Selon l'invention le problème est résolu par une méthode basée sur : 1) une amplification de cellules non transformées, notamment de cellules issues de biopsies de peau ; 2) un modèle mécanistique valable pour les cellules humaines quiescentes ; 3) des tests fonctionnels de la reconnaissance, de la réparation et de la signalisation des CDB valables quelle que soit la modalité thérapeutique.

L'invention est décrite dans les revendications annexées.

La description décrit un procédé pour caractériser la radiosensibilité cellulaire d'un échantillon cellulaire prelevé sur un patient envers un rayonnement ionisant, à partir de cellules prélévées sur ledit patient dans une zone non irradiée ou peu irradiée, dans lequel :

(i) on amplifie lesdites cellules prelévées, ces cellules amplifiées constituant « l'échantillon cellulaire » ;
(ii) on détermine sur ledit échantillon cellulaire le nombre moyen de foci nucléaires obtenus avec au moins deux des marqueurs pH2AX, pATM et MRE11 aux temps d'observation t (ces nombres moyens étant appelé respectivement $N_{pH2AX}(t)$, $N_{pATM}(t)$, $N_{MRE11}(t)$), lesdits temps d'observation t étant le temps t=0 min (appelé t0, état non irradié) et au moins un temps d'observation sélectionné parmi t = t1, t2, t3 et t4 après irradiation avec une dose absorbée D;
(iii) on détermine au moins un paramètres sélectionné dans le groupe formé par:

- le grade de sévérité de la réaction tissulaire post-radiothérapie selon la classification CTCAE, en utilisant au moins les nombres moyens $N_{pH2AX}(t)$ et $N_{pH2AX}(tx)$ où tx est soit t4 (préféré) ou t3 ;
- le groupe de radiosensibilité de l'échantillon, en utilisant au moins les nombres moyens $N_{pH2AX}(t)$, $N_{pATM}(t)$ et $N_{MRE11}(t)$ ;

et dans lequel procédé

- t4 est une valeur fixe qui représente le temps pour lequel le taux de cassures ADN atteint sa valeur résiduelle, et qui est avantageusement choisie entre 6 fois t3 et 8 fois t3, mais doit être dans ce cas être au moins de 12 heures, et de préférence comprise entre 12h et 48h, et qui est encore plus préférentiellement d'environ 24 heures ;
- t3 est une valeur fixe qui représente le temps au bout duquel environ 25% des CDB sont réparées dans des cellules témoins issues de patients radiorésistants, et qui est avantageusement choisie entre 3 fois t2 et 5 fois t2, mais doit dans ce cas être au moins de 2,5 heures et au plus de 6 heures, et est de préférence comprise entre 3 heures et 5 heures, et est encore plus préférentiellement environ 4 heures ;
- t2 est une valeur fixe qui représente le temps au bout duquel environ 50% des CDB sont réparées dans des cellules témoins issues de patients radiorésistants, et qui est avantageusement choisie entre 5 fois t1 et 7 fois t1, mais qui doit dans ce cas être au moins de 35 minutes et au plus de 90 minutes, et est de préférence compris entre 45 minutes et 75 minutes, et est encore plus préférentiellement environ 60 minutes ;
- t1 est une valeur fixe qui représente le temps au bout duquel le nombre de CDB reconnues atteint son maximum dans des cellules témoins issues de patients radiorésistants, et qui est avantageusement chosie entre 5 minutes et 15 minutes après l'arrêt de l'irradiation, de préférence entre 7,5 minutes et 12,5 minutes, et encore plus préférentiellement à environ 10 minutes.

Dans un mode de réalisation t1 est compris entre 8 minutes et 12 minutes, t2 est compris entre 50 minutes et 70 minutes, t3 est compris entre 3,5 heures et 4,5 heures, et t4 est compris entre 22 heures et 26 heures.
Dans un mode de réalisation on détermine en plus sur ledit échantillon cellulaire le nombre moyen de micronoyaux observés aux temps t pour 100 cellules [en %] (ce nombre moyen étant appelé $N_{MN}(t)$), les temps t étant au moins t0 (non irradié) et t4 après irradiation avec une dose absorbée D.
Ledit rayonnement ionisant est de nature thérapeutique. En particulier, la dose absorbée D est comprise entre 0,5 Gy et 4 Gy, de préférence comprise entre 1 Gy et 3 Gy, de préférence entre 1,7 Gy et 2,3 Gy, et est encore plus préférentiellement de 2 Gy.
Dans un mode de réalisation avantageux t1 est 10 minutes, t2 est 60 minutes, t3 est 4 heures, t4 est 24 heures, et D est 2 Gy.
Dans le cadre de la présente invention un paramètre pour caractériser la réaction cellulaire d'un patient envers un rayonnement ionisant est le grade de sévérité selon la classification CTCAE, exprimé comme paramètre sans dimension, et déterminé comme

$$ CTCAE = 5 - Max[N_{pATM}(t1) \, ; \, N_{pATM}(t2)]/10. $$

L'utilisation de cette formule selon l'invention permettant la détermination du grade de sévérité selon la classification CTCAE conduit à l'obtention d'un chiffre décimal. La valeur, selon l'invention, de la CTCAE finale obtenue est un chiffre entier correspondant à l'arrondi arithmétique de la valeur obtenue par calcul. La CTCAE déterminée selon l'invention et indiquée dans la demande correspond à ce chiffre ou à ce nombre entier.
En pratique, cette formule est particulièrement adaptée à la détermination du grade de sévérité de patients appartenant au groupe de radiosensibilité de type II (radiosensibilité modérée). Concernant les patients appartenant au groupe de radiosensibilité de type III (patients radiosensibles), la radiothérapie est prohibée.

Dans un mode de réalisation, on détermine le groupe de radiosensibilité de la manière suivante :

(a) on considère que l'échantillon est radiorésistant si $N_{pH2AX}(t4) < 2$ et $N_{pATM}(t1) > N_{pATM}(t2)$ et $N_{pATM}(t1) > 30$ et $A < 10$ et $B < 5$ et $C < 2$ ; sachant que :

$$C = N_{pH2AX}(t0) + N_{MN}(t0) ;$$

$$B = \% \text{ de gros noyaux (supérieurs à 150 } \mu m^2) \text{ à t0 ;}$$

$$A = N_{MRE11}(t0) + \text{Nbre de petit foci pH2AX/cellule à t0 ;}$$

(b) on considère que l'échantillon est très radiosensible si $(N_{pH2AX}(t4) > 8$ ou $N_{MN}(t4) > 24)$ ;

(c) on considère que l'échantillon présente une radiosensibilité modérée pour toutes les autres conditions.

Pour certains patients, la réparation de l'ADN peut être perturbée par une production incessante de cassures simple-brin de l'ADN (CSB) spontanées dû au phénomène d'hyperrecombinaison qui est généralement observé chez les patients prédisposés aux cancers. La surproduction spontanée de CSB peut avoir deux effets qui ne non pas contradictoires : à l'état spontané et par marquage pH2AX, des foci nucléaires plus petits que les foci pH2AX habituellement observés peuvent apparaître ; ils sont le reflet de la présence d'un grand nombre de CDB (phénomène de « petits focis »). De même, une surproduction de CSB peut conduire à une décondensation de la chromatine ce qui augmente la taille du noyau des cellules (taille généralement supérieur à 150 $\mu m^2$, corresdpondant au phénomène des « gros noyaux »). Ces deux phénomènes sont le reflèt d'une grande instabilité génomique.

La détermination de $N_{pH2AX}$, $N_{pATM}$ et/ou $N_{MRE11}$ fait avantageusement intervenir un test d'immunofluorescence.

Dans un mode de réalisation avantageux lesdites cellules témoins issues de patients radiorésistants ont été sélectionnées en tant que cellules montrant un taux de survie clonogénique *in vitro* supérieur à 55% après une irradiation avec une dose absorbée de 2 Gy.

Dans un autre mode de réalisation lesdites cellules témoins issues de patients radiorésistants ont été sélectionnées en tant que cellules prélevées sur des patients n'ayant pas montré de réactions tissulaires significatives pendant ou à la suite d'un traitement radiothérapeutique.

Le procédé décrit ci-dessus utilise au moins un échantillon de tissu sain, de préférence des fibroblastes. Ces derniers sont de préférence prélevés sur le tissu conjonctif du patient. Ce prélèvement peut être effectué par biopsie. Ainsi, dans un mode de réalisation avantageux lesdites cellules prélevées sont des cellules fibroblastiques issues d'une biopsie de peau d'un patient (prélevées typiquement selon une méthode connue sous le nom « punch dermatologique »). L'échantillon de tissu est cultivé dans un milieu de culture approprié.

La première étape du procédé décrit ci-dessus qui suit le prélèvement des cellules (à savoir dans le mode de réalisation préféré l'établissemement de la biopsie en lignée fibroblastique) consiste à caractériser l'état spontané de l'ADN (état à t0), c'est-à-dire sans irrradiation. Cette étape peut comporter notamment l'examen de la taille des noyaux, la présence de micronoyaux, des éventuels corps apoptotiques spontanés et de cellules multilésées : on observe les cellules sous un microscope à fluorescence. En utilisant le colorant DAPI (4'6'-diamidino-2-phénylindole, n° CAS 28718-90-3 pour le dihydrochlorure) on détermine le taux de micronoyaux pour 100 cellules, qui est un indicateur de l'instabilité génomique. On détermine également les corps apoptotiques. On détermine également la population de noyaux anormalement gros, dont la présence indique la décondensation de la chromatine.

Ledit rayonnement ionisant est défini par sa dose absorbée (paramètre appelé D et exprimé en Gray). Dans le cadre de la présente invention la dose absorbée D est comprise entre 0,5 Gy et 4 Gy, de préférence comprise entre 1 Gy et 3 Gy, de préférence entre 1,7 Gy et 2,3 Gy, et est encore plus préférentiellement de 2 Gy. Ces zones correspondent typiquement à une séance individuelle de traitement radiothérapeutique, le nombre de séances dépendant de la localisation, du type et du stade d'avancement de la tumeur.

Il est essentiel pour la méthode décrite ci-dessus que toutes les valeurs de temps t1, t2, t3 et t4 soient définies en début d'une série d'essais (i.e. au moins pour un patient donné, et de préférence pour une pluralité de patients afin de calibrer la méthode par rapport à un ensemble d'observations statistiquement significatifs) et soient les mêmes pour toutes les déterminations de tous les paramètres qui se réfèrent à ces intervalles de temps.

Dans le procédé décrit ci-dessus, t1 est avantageusement compris entre 8 minutes et 12 minutes, et/ou t2 est avantageusement compris entre 50 minutes et 70 minutes, et/ou t3 est avantageusement compris entre 3,5 heures et 4,5 heures, et/ou t4 est avantageusement compris entre 22 heures et 26 heures ; de préférence toutes ses quatre conditions sont remplies.

Dans une variante du procédé qui est particulièrement intéressante et facile à standardiser, t1 est 10 minutes, t2 est 60 minutes, t3 est 4 heures, t4 est 24 heures, et D est 2 Gy.

Les cellules témoins issues de patients radiorésistants peuvent être prélevées sur des patients sélectionnés à partir d'un examen clinique comme patients n'ayant pas montré de réactions tissulaires significatives pendant ou à la suite d'un traitement radiothérapeutique. Elles peuvent également être sélectionnées en tant que cellules montrant un taux de survie clonogénique *in vitro* supérieur à 55% après une irradiation avec une dose absorbée de 2 Gy.

Nous décrivons ici un mode de réalisation typique.

On observe les cellules avec les marqueur pH2AX, pATM et/ou MRE11 aux temps d'observation t (ces nombres moyens étant appelés respectivement $N_{pH2AX}(t)$, $N_{pATM}(t)$ et $N_{MRE11}(t)$) et au moins un temps d'observation sélectionné parmi t=t1, t2 , t3 et t4 après irradiation avec une dose absorbée D. Dans un mode de réalisation on détermine le nombre de foci avec le marqueur pH2AX et la présence de cellules multilésées. On note la localisation de la protéine pATM et celle de la protéine MRE11 (nucléaire ou cytoplasmique).

Cette première étape permet d'identifier une éventuelle instabilité génomique à l'état spontanée.

La seconde étape du procédé selon l'invention comprend l'irradiation avec la dose absorbée D voulue (par exemple 2 Gy) et l'évaluation de la réponse cellulaire au rayonnement ionisant.

a) Dans un premier mode de réalisation, on étudie la réparation des CDB radioinduites par suture, qui est le mode majoritaire de leur réparation. On détermine le nombre de foci pH2AX par cellule à t4 et optionnellement aussi à t1, t2 et possiblement aussi à t3 ; la détermination à t3 permet de consolider la définition de l'allure des cinétiques de t1 à t4. Dans un mode de réalisation avantageux, après la durée t4 on détermine également le taux de micronoyaux pour en déduire le taux de micronoyaux radioinduits. Cela permet d'estimer la radiosensibilité selon l'importance des CDB non réparées.

b) Dans un deuxième mode de réalisation on approfondit l'étude de la réponse cellulaire au rayonnement ionisant à travers la mesure de la fonctionnalité de l'activité kinase ATM-dépendante. On sait que dans les cellules radiorésistantes témoin, les formes phosphorylées de la protéine ATM (pATM) sont cytoplasmiques à l'état spontané. La demanderesse a découvert qu'à l'état irradié, elles ont tendance à devenir nucléaires. Une fois passées dans le noyau, les formes pATM activent les mécanismes de réparation par suture et inhibent la voie de réparation fautive dépendante de MRE11.

A titre d'exemple, si après l'irradiation (par exemple avec une dose absorbée de 2 Gy) les formes pATM montent une localisation cytoplasmique, on en conclut que les formes pATM ne passent pas ou ne peuvent pas passer normalement du cytoplasme au noyau. Cela peut être causé par une mutation d'ATM ou de tout autre partenaire protéine d'ATM qui l'aiderait à passer dans le noyau après irradiation : en tout cas, cela indique une radiosensibilité significative.

Cette détermination optionnelle de la localisation de la protéine pATM est effectuée au moins à t1 et t2, et optionnellement aussi à t3 et t4.

c) dans un troisième mode de réalisation pouvant être combiné avec les précédents, on approfondit l'étude de la réponse cellulaire au rayonnement ionisant à travers la voie MRE11-dépendante. En plus de la voie majeure de réparation par suture dont la capacité est quantifiable par immunofluorescence pH2AX, la demanderesse a identifié une autre voie de réparation, alternative à la suture et qui est susceptible de la remplacer en cas de déficience: c'est la réparation par recombinaison MRE11 dépendante. Sa capacité est quantifiable par l'étude cinétique de l'immunofluorescence des foci MRE11. Cette mesure est effectuée au moins à t1, t2 et t3, et optionnellement aussi à t4. Selon les constatations de la demanderesse, dans les lignées témoins radiorésistantes, MRE11 est cytoplasmique et le nombre de foci MRE11 est très faible jusqu'à 4 heures après une dose 2 Gy (typiquement $7 \pm 2$ foci MRE11) ; le marquage devient cytoplasmique environ 24 heures après l'irradiation.

Dans une dernière étape on procède à l'évaluation des résultats en calculant les scores afin de prédire l'état d'endommagement radioinduit et/ou la radiosensibilité du patient, et en particulier la CTCAE qui lui est propre.

**Description des figures**

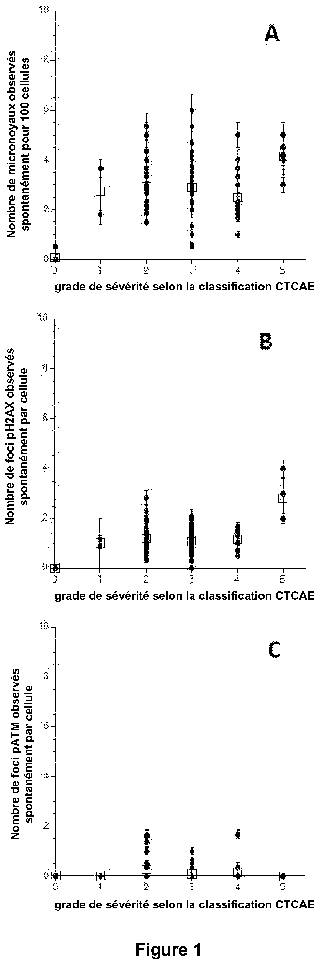

Les figures 1 (a), (b) et (c) montrent respectivement l'évolution du nombre de foci des micronoyaux (a), des marqueurs pH2AX (b) et pATM (c) issus de cellules non irradiées en fonction du grade de sévérité selon la classification CTCAE. Les micronoyaux, les foci pH2AX et pATM issus de cellules non irradiées ne sont pas prédictives de la radiosensibilité.

Deux échelles différentes de gravité de réactions tissulaires co-existent : la classification CTCAE et la classification RTOC.

La classification dite CTCAE (Common Terminology Criteria for Adverse Events, appelée en français « Critères d'évaluation de la morbidité selon la classification du National Cancer Institute»), éditée en 2006 par le National Cancer Institute des Etats Unis d'Amérique, est une terminologie descriptive des évènements indésirables (en particulier des effets secondaires) en thérapie du cancer.

Un événement indésirable correspond à tout signe défavorable et involontaire, symptôme ou maladie temporellement associé à l'utilisation d'un traitement médical ou d'une procédure qui peut ou non être considéré comme lié au traitement ou à la procédure médicale. Un événement indésirable est une représentation unique d'un événement spécifique utilisé pour la documentation médicale et lors d'analyses scientifiques.

La CTCAE fournie une brève définition de chaque événement indésirable pour clarifier le sens de l'événement indésirable. Cette échelle, valable pour d'autres stress génotoxique (ex : blessures causées par le feu) est particulièrement utilisée en radiothérapie.

Le grade se réfère à la sévérité de l'événement indésirable. La CTCAE affiche 5 grades de sévérité (de 1 à 5) présentant des descriptions cliniques uniques de sévérité pour chaque événement indésirable et décrits dans le tableau 1 ci-après. Chaque grade de sévérité est défini par des réactions tissulaires précises.

Tableau 1 : dernière version de l'échelle CTCAE éditée par le National Cancer Institute des Etats Unis d'Amérique le 14 juin 2010

| grade 1 | Sévérité légère; symptômes asymptomatiques ou légers; observations cliniques ou diagnostiques seules; intervention non indiquée |
|---------|------|
| grade 2 | Sévérité modérée; intervention minimale, intervention locale ou non invasive indiquée; évènement limitant les activités de la vie quotidienne instrumentées et adaptées à l'âge (préparation du repas, faire les courses, utilisation du téléphone...) |
| grade 3 | Sévérité grave ou médicalement significative, mais ne mettant pas immédiatement la vie en danger; hospitalisation ou prolongation de l'hospitalisation indiquée; évènement invalidant; évènement limitant les activités de soins de la vie quotidienne (bains, s'habiller, se nourrir, utilisation des toilettes, prise de médicaments, et ne pas être alité) |
| grade 4 | Conséquences potentiellement mortelles; intervention urgente indiquée |
| grade 5 | Décès lié à l'événement indésirable |

A ces 5 grades, est ajouté un grade 0 correspondant à une absence d'effet tissulaire. La classification historique dite RTOG, proposée par le Radiation Therapy Oncology Group (RTOC) en 1984 couvre pratiquement tous les types de toxicités tardives de la radiothérapie.

Toutefois, la classification RTOG n'est pas applicable pour certains types de cancer sachant que la classification CTCAE, elle, est employée pour tout type de cancer.

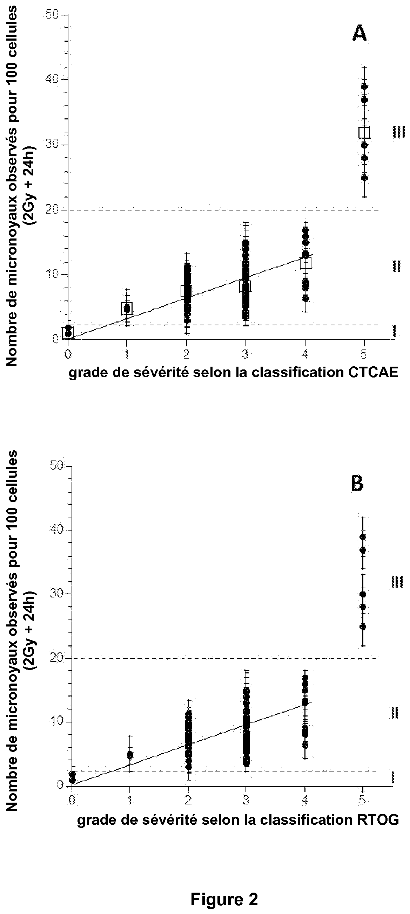

Les figures 2 (a) et 2 (b) présentent l'évolution du nombre de micronoyaux 24 h après irradiation en fonction des grades de sévérité CTCAE (figure 2 (a)) ou RTOG (figures 2 (b)). Les micronoyaux sont marqués avec le marqueur fluorescent DAPI puis quantifiés par l'analyse du signal de fluorescence. Le groupe de radiosensibilité (I, II, III) est indiqué en caractères romains sur la figure 2.

Le nombre de micronoyaux observé 24 h après irradiation permet uniquement de prédire les radiosensibilités de groupe III.

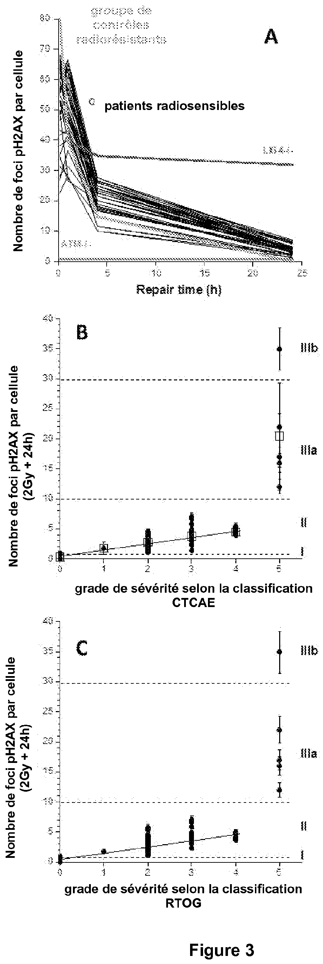

Les figures 3 (a), (b) et (c) montrent l'évolution du nombre de foci pH2AX 24 h après irradiation en fonction des grades de sévérité CTCAE (figure 3 (b)) ou RTOG (figure 3 (c)). La figure 3 (a) présente les cinétiques du nombre moyen de foci obtenus avec le marqueur pH2AX au cours du temps.

Le nombre de foci pH2AX obtenu 24 h après irradiation en fonction des grades de sévérité CTCAE ou RTOG (2 échelles différentes de gravité de réactions tissulaires) permettent de prédire uniquement les radiosensibilités de groupe I, II ou III mais pas les grades de sévérité.

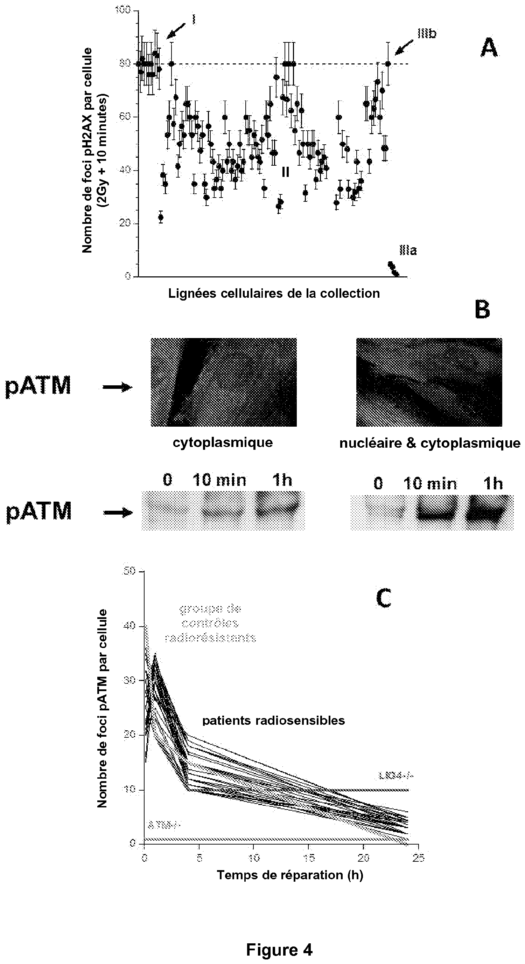

La figure 4 (A) représente le nombre moyen de foci obtenus avec le marqueur pH2AX 10 minutes après une irradiation avec 2 Gy pour toutes les lignées cellulaires d'une collection d'échantillons de patients (fibroblastes de peau), la ligne pointillée indiquant l'incidence de CDB normale qui est de 40 CDB par Gy par cellule.

La figure 4 (A) montre que toutes les cellules issues de patients ayant une radiosensibilité de groupe II sont caractérisées par moins de foci pH2AX (de cassures double-brin de l'ADN (CDB)) qu'attendus après 2Gy. Ceci s'explique par le fait que les CDB sont insuffisamment reconnues.

La figure 4 (B) représente l'expression des pATM dans le cytoplasme et le noyau à différents temps (0 minute, 10 minutes et 1 h) suite à une irradiation de 2 Gy. Les données d'immunofluorescence correspondantes sont présentées pour des cellules non irradiées et pour des cellules irradiées avec 2 Gy à 10 minutes post-irradiation.

Les données présentées dans la figure 4 (B), relatives aux nombres de foci pATM suggèrent un « transit cyto-nucléaire » de l'ATM.

La figure 4 (C) présente les cinétiques du nombre moyen de foci obtenus avec le marqueur pATM au cours du temps à partir de cellules de la collection. Par commodité, les barres d'erreur relatives aux mesures effectuées à 10 min, 1 h, 4 h et 24 h ont été omises. Pour les figures 4 (A) et 4 (C), chaque point représente la moyenne de trois répétitions indépendantes et les barres d'erreur représentent l'écart-type pour chaque catégorie.

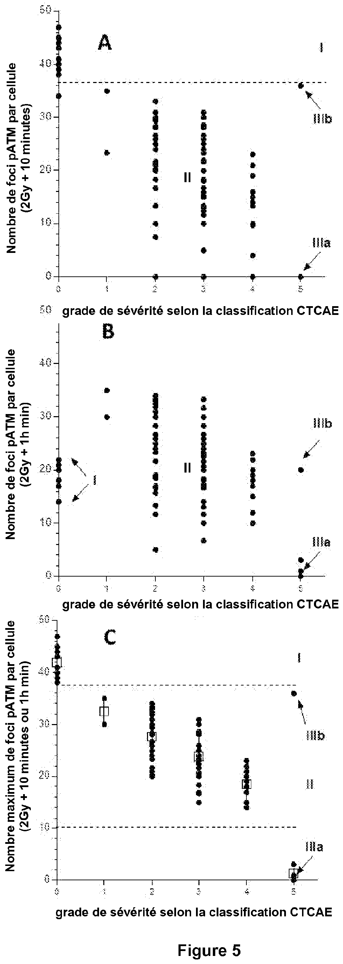

La figure 5 montre l'évolution du nombre de foci pATM en fonction des grades de sévérité CTCAE après 10 minutes (figure 5 (A)) et 1 h suite à une irradiation de 2 Gy (figure 5 (B)). La figure 5 (C) représente le maximum du nombre de foci pATM entre les 2 valeurs obtenues à 10 minutes et 24 h après une irradiation de 2 Gy en fonction des grades de sévérité CTCAE, et précédemment présentées en figure 5(A) et 5(B) respectivement. La figure 5 (B) présente un grade 0, c'est-à-dire une absence d'effet tissulaire.

La figure 5 (C) montre pour une centaine de patients le lien existant entre ces paramètres radiobiologiques et les grades de sévérité selon la classification CTCAE. La figure 5 (C) représente ainsi la validation clinique de la corrélation existante entre les grades de sévérité CTCAE et le maximum du nombre de foci pATM entre les 2 valeurs obtenues 10 minutes et 1 h après irradiation de 2 Gy.

Les groupes de radiosensibilité (I, II, IIIa et IIIb) sont indiqués en caractères romains sur la figure 5. Pour les figures 5 (A), 5 (B) et 5 (C), chaque point représente la moyenne de trois répétitions indépendantes, et ce, pour chaque catégorie.

Le nombre de foci pATM maximal entre (2 Gy + 10 min) ou (2 Gy + 1h) permet de prédire tous les groupes ainsi que le grade de sévérité de la réaction.

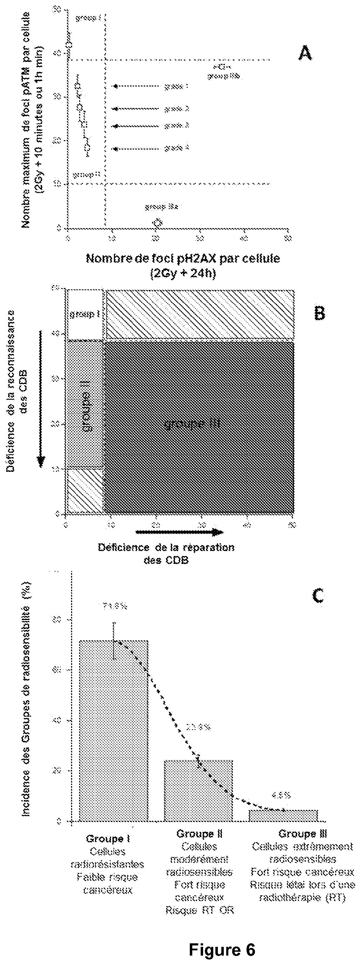

La figure 6 (A) représente le nombre maximum de foci obtenus avec le marqueur pATM en fonction du nombre de foci pH2AX, précédemment représenté en figures 3 (B) et 5 (C) respectivement.

La figure 6 (B) reprend les données présentées en figure 6 (A) et montre des zones de confiance bien définies représentant les différents groupes de radiosensibilité humaine (Groupe I, Groupe II et Groupe III). La radiosensibilité est déterminée par la reconnaissance et la réparation des cassures double-brin.

La figure 6 (C) présente l'incidence des groupes pour chaque type de groupes. En considérant que la probabilité d'appartenance à un groupe donné est proportionnelle à l'inverse des zones de confiance correspondants, la fréquence normalisée de chaque groupe est représentée sur la figure 6 (C) par des barres. La ligne en pointillé correspond à la gaussienne produisant le meilleur ajustement des données ($r = 0,9$).

## Description de l'invention

### A. Définitions générales

Les termes « endommagement radioinduit », « radioinduit », « radiosensibilité, « radiorésistance », « radiotoxicité », « radiothérapie » se réfèrent tous à un rayonnement ionisant, notamment à un rayonnement de type particules, tel que constitué par des particules de type alpha ($\alpha$) ou béta ($\beta$), ou encore à un rayonnement électromagnétique à haute énergie, notamment de type gamma ($\gamma$) ou X.

Le terme transit cyto-nucléaire d'ATM décrit la translocation qu'effectue la protéine ATM en passant du cytoplasme au noyau, notamment après irradiation.

### B. Description détaillée

Nous décrivons ici un mode de réalisation avec plusieurs variantes qui convient pour un patient humain.

#### 1. Préparation de l'essai

Avant tout prélèvement de cellules et avant toute manipulation des cellules prelévées, les opérateurs respectifs (appartenant par exemple à un laboratoire d'analyses cytologiques) sont informés (typiquement par le médecin) du statut d'infection éventuelle du patient par HIV ou hépatite C pour que les opérateurs puissent prendre les mesures appropriées de sécurité biologique accrue lors du prélèvement, de la manipulation et de la gestion de la culture cellulaire.

Puis, l'opérateur prélève au patient un échantllon cellulaire. De préférence il lui prélève par biopsie un échantillon de peau ; ce prélèvement peut se faire avantageusement selon une méthode connue sous le nom « punch dermatologique ». L'échantillon cellulaire est placé dans du milieu DMEM+20% sérum de veau fetal stérile. L'échantillon est transféré sans délai dans un laboratoire spécialisé, sachant que l'échantillon ne doit pas rester plus de 38 h à la température ambiante.

Dès réception, l'échantillon cellulaire (typiquement la biopsie) est établie sous la forme d'une lignée cellulaire amplifiable sans agent de transformation virale ou chimique suivant une procédure ancillaire et bien connue des laboratoires de culture, comme le souligne la publication d'Elkin M. et al. « The radiobiology of cultured mammalian cells », Gordon and Breach (1967). Dès que le nombre de cellules est suffisant (1 - 3 semaines), les premières expériences sont réalisées en utilisant le procédé selon l'invention. Les cellules sont ensemencées sur lamelles de verre dans des boîtes de Petri. Une partie de ces lamelles sont irradiées sur un irradiateur médical suivant une dosimétrie certifiée avec une dose absorbée D (par exemple 2 Gy). Une autre partie n'est pas irradié ; elle repésente l'état spontané (dose absorbée 0 Gy).

L'irradiation peut être effectuée par exemple avec un accélérateur médical qui délivre des photons de 6 MV avec un débit de dose absorbée de 3 Gy min$^{-1}$. Après l'irradiation et pour subir les temps de réparation mentionnés ci-dessous, les cellules restent dans l'incubateur de culture à 37°C.

Pour les cellules irradiées, on acquiert des caractéristiques correpondant à l'état radioinduit après plusieurs temps de réparation (temps de réparation post-irradiation). On acquiert de préférence au moins deux et encore plus préférentiellement au moins trois points, à savoir : t1, t2, t3 et t4. Lesdites caractéristiques sont représentées par les foci correspondant au marqueur pH2AX.

Les cellules sur lamelles de verre sont ensuite fixées, lysées et hybridées. La procédure suivante, connue en tant que telle (voir la publication citée de Bodgi et al.), peut être utilisée : les cellules ont été fixés dans 3% paraformaldéhyde et 2% sucrose pendant 15 minutes à température ambiante et perméabilisées dans 20 mM solution tampon HEPES (acide 4-(2-hydroxyéthyl)-1-pipérazine éthane sulfonique) à pH 7,4, 50 mM NaCl, 3 mM MgCl$_2$, 300 mM sucrose, 0,5% Triton X-100 (un surfactant non-ionique de formule $t$-Oct-C$_6$H$_4$-(OCH$_2$CH$_2$)$_x$OH avec x= 9-10, CAS N° 9002-93-1, fourni par Sigma Aldrich) pendant 3 minutes. Puis les lamelles de couverture ont été lavées dans du tampon phosphate salin (connu sous le sigle PBS) avant coloration immunologique. L'incubation a eu lieu pendant 40 min à 37°C dans du PBS additioné de 2% d'albumine de sérum bovin (connu sous le sigle BSA ou fraction V, fourni par Sigma Aldrich) et a été suivi par un lavage au PBS. Les anticorps primaires anti-pH2AX ont été utilisés à une concentration de 1 :800, les autres anticorps primaires à 1 :100. Les incubations avec des anticorps secondaires FITC anti-souri ou TRITC anti-lapin (1:100, fournis par Sigma Aldrich) ont été effectuées à 37°C dans du BSA à 2% pendant 20 minutes. On a traité des lamelles de verre avec du Vectashield™ contenant du DAPI (4,6-Diamidino-2-phénylindole) pour marquer le noyau. La coloration avec du DAPI pemet aussi, de manière indirecte, la détermination du nombre de cellules en phase G$_1$ (noyaux avec coloration DAPI homogène), en phase S (noyaux avec de nombreux foci pH2AX), en phase G$_2$ (noyaux avec coloration DAPI hétérogène) et métaphases (chromosomes visibles).

L'acquisition des résultats est effectuée à partir de ces lamelles sur microscope à immunofluorescence (modèle Olympus par exemple). La lecture peut être directe (typiquement par comptage des foci sur au moins 50 cellules en G$_0$/G$_1$ pour chaque point) ou par logiciel d'analyse d'image dédié, ou encore sur un microscope automatisé ; de préférence les méthodes par logiciel ou microscope automatisé sont calibrées avec des déterminations manuelles.

Afin d'obtenir des résultats d'une fiabilité statistique suffisante pour servir comme base d'un diagnostic, au moins 3 series d'expériences (irradiation) indépendantes sont effectuées et la moyenne de chacun des nombres de foci pour les temps définis est calculée.

## 2. Détermination des paramètres biologiques et cliniques

### 2.1 Généralités et marqueurs utilisés

L'invention est basée entre autres sur l'utilisation de données acquises pour au moins deux des trois marqueurs pH2AX, pATM et MRE11 sur des cellules non-irradiées (état spontané) et irradiées (état radioinduit). La méthode est basée sur l'étude cinétique du marquage par ce marqueur en fonction de la durée de réparation : on marque les échantillons après un laps de temps déterminé à compter de l'arrêt de l'irradiation, et on étudie leur immunofluorescence. On peut mesurer les courbes cinétiques complètes, par exemple représentées par 5 points se situant avantageusement à t0, t1 (de préférence 10 minutes), t2 (de préférence 1h), t3 (de préférence 4h) et t4 (de préférence 24h), sachant que t0 correspond à l'état avant irradiation (état spontané).

Mais la demanderesse s'est rendue compte que certains points (correspondant à certains temps de réparation) sont plus importants que d'autres, et que certains points ne sont pas prédictifs. Grâce à la sélection judicieuse des paramètres déterminés à des temps donnés, on peut ainsi diminuer le nombre de mesures et donc diminuer le coût global du diagnostic, sans diminuer le pouvoir prédictif de la méthode. C'est cette méthode simplifiée qui constitue la base de la méthode prédictive selon l'invention.

Les moyennes de chaque point et chaque dose avec chaque marqueurs sont calculées avec les erreurs écart-types de la moyenne (SEM) vu que l'échantillonnage est n=3 (pas d'écart-type gaussien de type standard error SE).

(i) pH2AX désigne les formes phosphorylées en sérine 439 du variant X de l'histone H2AX qui marque, selon les constatations de la demanderesse, le nombre de cassures double-brin de l'ADN (CDB) qui sont reconnues par le mode de réparation majoritaire et fidèle, la suture. Le marqueur pH2AX est essentiellement nucléaire sous forme uniquement de foci nucléaire et seuls le nombre et la taille des foci seront analysés.

(ii) pATM désigne les formes phosphorylées en sérine 1981 de la protéine kinase ATM. Selon les constatation de la demanderese, ATM passe du cytoplasme au noyau après irradiation en conditions normales (statut radiorésistant). Les formes pATM se concentrent principalement dans le cytoplasme puis marquent des sites de CDB. Le marqueur pATM se distingue par une localisation qui peut être cytoplasmique homogène (pas de foci cytoplasmique) sans foci nucléaire, uniquement nucléaire sous forme uniquement de foci nucléaires (pas de localisation nucléaire homogène), soit cytoplasmique et foci nucléaire.

(iii) MRE11 est une endonucléase qui casse l'ADN. Selon les constatations de la demanderesse, MRE11 marque les CDB mal réparées quand le processus de réparation est finalisé. Le marqueur MRE11 peut être soit cytoplasmique sans foci, soit cytoplasmique et nucléaire sans foci, soit cytoplasmique et nucléaire avec foci.

La contrecoloration au DAPI (un marqueur d'ADN connu de l'homme du métier) permet de localiser le noyau pour situer la localisation cytoplasmique ou nucléaire (cette répartition étant modifiée pour MRE11 et pATM sous l'influence de la radiation ionisante), pour quantifier les micronoyaux, les corps apoptotiques et la taille des noyaux qui sont des marqueurs cellulaires complémentaires aux données sur les foci.

2.2 Paramètres biologiques

On définit et détermine comme indiqué :

- $N_{pH2AX}(t)$, $N_{pATM}(t)$, $N_{MRE11}(t)$ les nombres moyens de foci nucléaires obtenus avec les marqueurs pH2AX, pATM, et MRE11 aux temps d'observations t0 (non irradié) ou t1, t2, t3, t4 après irradiation (dose absorbée: 2 Gy), sachant que la détermination du paramètre $N_{pH2AX}(t)$ est obligatoire dans le cadre de la méthode selon l'invention, alors que celle des autres paramètres $N_{pATM}(t)$ et $N_{MRE11}(t)$ est facultative mais avantageuse ;
- $N_{MN}(t)$ le nombre de micronoyaux observés spontanément (à t = t0, i.e. sans irradiation) ou à t = t4 après irradiation avec une dose absorbée de 2 Gy pour 100 cellules (en %).

2.3 Evaluation prédictive

Elle vise la prédiction de paramètres cliniques ou radiothérapiques à partir des données biologiques mesurées. Deux niveaux de diagnostic sont proposés :

a) un diagnostic quantitatif directement issu de la valeur mathématique des scores ou de formules mathématiques reliant les scores ; celui-ci concerne les deux critères suivants :

(i) La classification du patient dans un groupe I, II ou III (critère appelé GROUPE):
La définition des groupes de radiosensibilité (GROUPE) aide le médecin à déterminer à partir des scores selon l'invention et du tableau clinique du patient des analogies avec des syndromes génétiques connus. Ces groupes ont été définis dans la publication de Joubert et al qui a déjà été citée.

• Selon l'invention, on considère que :

Si $N_{pH2AX}(t4) < 2$ et
si $N_{pATM}(t1) > N_{pATM}(t2)$ et
si $N_{pATM}(t1) > 30$ et
si A < 10 et
si B < 5 et
si C < 2

sachant que :

$$C = N_{pH2AX}(t0) + N_{MN}(t0) \; ;$$

$$B = \text{\% de gros noyaux (supérieurs à 150 μm}^2\text{) à t0 ;}$$

$$A = N_{MRE11}(t0) + \text{nombre de petit foci pH2AX/cellule à t0 ;}$$

alors le groupe de radiosensibilité (critère GROUPE) est considéré comme « Groupe I » : ces cellules sont radiorésistantes.

- Si $(N_{pH2AX}(t4) > 8$ ou $N_{MN}(t4) > 24)$
alors on considère que le groupe de radiosensibilité (critère GROUPE) est considéré comme « Groupe III » : ces cellules sont très radiosensibles.
- Pour toutes les autres conditions, on considère que le critère GROUPE est « Groupe II » : ces cellules montrent une radiosensibilité modérée.

(ii) Le grade de gravité de la réaction tissulaire attendue suivant la classification CTCAE aiguë (critère appelé CTCAE).

La classification dite CTCAE (Common Terminology Criteria for Adverse Events, appelée en français « Critères d'évaluation de la morbidité selon la classification du National Cancer Institute»), éditée en 2006 par le National Cancer Institute des Etats Unis d'Amérique, est une terminologie descriptive des évènements indésirables (en particulier des effets secondaires) en thérapie du cancer.

Selon l'invention, ce grade est déterminé selon l'équation suivante :

$$CTCAE = 5 - [\max(N_{pATM}(t1); N_{pATM}(t2)]/10$$

b) un diagnostic plus qualitatif, influencé par le diagnostic quantitatif mais qui tient compte d'éventuels éléments cliniques portés à la connaissance du praticien.

### 2.5 Les niveaux et méthodes d'analyse

Dans un premier mode de réalisation, on relie tous les paramètres mesurés expérimentalement mathématiquement entre eux (pour certains). En cas de conflit entre scores on repète certaines expériences ou détermination, ou on en ajoute d'autres. Le niveau de certains paramètres seuls suffisaient à établir un score et un diagnostic.

Dans un deuxième mode de réalisation, on tient compte du constat que certains points ne sont pas prédictifs pour aucun score : tel est le cas par exemple des points $N_{pH2AX}(t3)$, $N_{pATM}(t3$ et $t4)$. C'est pourquoi on peut envisager une analyse restreinte où seulement les points t0, t1, t2 et t4 sont utilisés pour pH2AX, les points t0, t1 et t2 pour pATM, et les points t0, t1, t2, et t3 pour MRE11.

Cette méthode prédictive de détermination du grade de sévérité selon la classification CTCAE a été appliquée sur des tissus sains qui sont en majorité du tissu conjonctif de type fibroblastique. Elle peut être appliquée à tout type de cancer. La corrélation obtenue entre pATM et CTCAE permettant de déterminer le grade de sévérité selon la classification CTCAE de patients (cf. figure 5 (C)), et présentée dans la demande, a été validée sur des patients atteints de cancer du sein, du mediastin, de tout type de cancer ORL, de neuroblastome, de la prostate, de l'utérus, de l'ovaire et du rectum.

La méthode de détermination de la radiosensibilité de l'échantillon d'un patient selon l'invention a été appliquée sur des tissus du type cellules tumorales, cellules de patients atteints de cancer du sein, de la prostate, de l'utérus, de l'ovaire, du rectum, de tout type de cancer ORL, de glioblastome, de neuroblastome, de retinoblastome, d'osteosarcome et de melanome.

L'invention est illustrée ci-dessous par des exemples qui cependant ne limitent aucunement l'invention. Ces exemples portent sur l'analyse de lignées cellulaires de patients permettant la détermination du grade de sévérité et du groupe de radiosensibilité auquel appartient le patient.

**EXEMPLES** :

### 1. Préparation de l'essai

Un échantillon cellulaire de peau d'un patient a été prélevé par biopsie via la méthode de « punch dermatologique » connue de l'homme du métier. L'échantillon cellulaire a ensuite été placé dans du milieu DMEM+20% sérum de veau fetal stérile. L'échantillon cellulaire a ensuite été transféré sans délai dans un laboratoire spécialisé, afin que l'échantillon ne reste pas plus de 38 h à la température ambiante.

Dès réception, l'échantillon cellulaire issu de la biopsie a été établi sous la forme d'une lignée cellulaire amplifiable suivant une procédure bien connue des laboratoires de culture et de l'homme du métier : en utilisant notamment la dispersion trypsique les cellules sont à nouveau diluée dans du milieu renouvelé et ainsi de suite jusqu'à obtention du nombre de cellules souhaité. Après obtention d'un nombre de cellules suffisant, (généralement au bout d'une à 3 semaines), les premières expériences ont été réalisées en utilisant le procédé selon l'invention. Les cellules ont été ensemencées sur lamelles de verre dans des boîtes de Pétri. Une partie de ces lamelles ont été irradiées sur un irradiateur médical suivant une dosimétrie certifiée avec une dose absorbée D de 2 Gy. Une autre partie n'a pas été irradiée, cette partie représentant l'état spontané (dose absorbée 0 Gy).

2. Détermination du nombre de foci pH2AX, MRE11 et pATM à l'état spontané et après 10 min (t1), 1h (t2), 4h (t3) et 24h (t4)) de réparation post-irradiation avec une dose absorbée de 2Gy, du nombre de micronoyaux $N_{MN}(t)$ observés à l'état spontané et après 24h de temps de réparation après irradiation avec une dose absorbée de 2 Gy pour 100 cellules (en %), du nombre de petit foci pH2AX par cellule à à l'état spontané et du pourcentage de gros noyaux (supérieurs à 150 $\mu m^2$) à t0.

Pour les cellules ayant subi l'irradiation, l'irradiation a été effectuée avec un accélérateur médical qui délivre des photons de 6 MV avec un débit de dose absorbée de 3 Gy min$^{-1}$.

Après irradiation avec une dose absorbée de 2 Gy, les cellules ont été conservées dans l'incubateur de culture à 37°C. Pour les cellules irradiées (état radioinduit), les échantillons ont été marqués après un lapse de temps déterminé (temps de réparation post-irradiation), à savoir : 10 min (t1), 1h (t2), 4h (t3) et 24h (t4) à compter de l'arrêt de l'irradiation, et on a acquis le nombre moyen de foci nucléaires obtenus avec les marqueurs pH2AX, MRE11 et pATM à ces différents temps de réparation post-irradiation (10 min (t1), 1h (t2), 4h (t3) et 24h (t4)). Le nombre de micronoyaux $N_{MN}(t)$ observés après 24h de temps de réparation après irradiation avec une dose absorbée de 2 Gy pour 100 cellules (en %) a aussi été déterminé via l'analyse de l'immunofluorescence de ces échantillons.

Pour les cellules non-irradiées (état spontané, i.e. à t0), on a acquis le nombre moyen de foci pH2AX à l'état spontané, le nombre de petit foci pH2AX par cellule à l'état spontané, le pourcentage de gros noyaux (supérieurs à 150 $\mu m^2$) à t0 et le nombre de micronoyaux observés spontanément via l'analyse de l'immunofluorescence de ces cellules.

Les cellules non-irradiées et les cellules ayant subi l'irradiation ont ensuite été fixées, lysées et hybridées sur des lamelles de verre. Les cellules ont été fixées dans 3% paraformaldéhyde et 2% sucrose pendant 15 minutes à température ambiante et perméabilisées dans 20 mM solution tampon HEPES (acide 4-(2-hydroxyéthyl)-1-pipérazine éthane sulfonique) à pH 7,4, 50 mM NaCl, 3 mM MgCl2, 300 mM sucrose, 0,5% Triton X-100 (un surfactant non-ionique de formule t-Oct-C6H4-(OCH2CH2)xOH avec x= 9-10, CAS N° 9002-93-1, fourni par Sigma Aldrich) pendant 3 minutes. Puis les lamelles de couverture ont été lavées dans du tampon phosphate salin (connu sous le sigle PBS) avant coloration immunologique. L'incubation a eu lieu pendant 40 min à 37°C dans du PBS additionné de 2% d'albumine de sérum bovin (connu sous le sigle BSA ou fraction V, fourni par Sigma Aldrich) et a été suivie par un lavage au PBS. Les anticorps primaires anti-pH2AX ont été utilisés à une concentration de 1 :800, les autres anticorps primaires à 1 :100. Les incubations avec des anticorps secondaires FITC anti-souri ou TRITC anti-lapin (1:100, fournis par Sigma Aldrich) ont été effectuées à 37°C dans du BSA à 2% pendant 20 minutes.

Les lamelles de verre ont ensuite été traitées avec du Vectashield™ contenant du DAPI (4,6-Diamidino-2-phénylindole) pour marquer le noyau. La coloration avec du DAPI a permis de manière indirecte, la détermination du nombre de cellules en phase de quiescence G0/G1 (noyaux avec coloration DAPI homogène), en phase de synthèse S (noyaux avec de nombreux foci pH2AX), en phase de quiescence G2 (noyaux avec coloration DAPI hétérogène) et en phase de mitose M (chromosomes visibles). La contrecoloration au DAPI a permis notamment de localiser le noyau pour situer sa localisation cytoplasmique ou nucléaire, et a permis ainsi de quantifier les micronoyaux présents.

L'acquisition des résultats a été effectuée à partir de ces lamelles sur microscope à immunofluorescence (modèle Olympus). La lecture a été réalisée de manière directe par comptage des foci obtenus avec les différents marqueurs pH2AX, pATM et MRE11 sur au moins 50 cellules en G0/G1 pour chaque point et par logiciel d'analyse d'image dédié (imageJ).

Afin d'obtenir des résultats d'une fiabilité statistique suffisante pour servir comme base d'un diagnostic, 3 séries d'irradiations indépendantes ont été effectuées. La moyenne et les erreurs écart-types de la moyenne (SEM) de chacun des nombres de foci à l'état spontané (t0), après 10 min (t1), 1h (t2), 4h (t3) et 24h (t4)) de réparation post-irradiation ont été calculées et présentées dans le tableau ci-après (cf. tableau 2), et ce, pour différents échantillons cellulaires de peau de patients.

Tableau 2 : Détermination du grade de sévérité et de la radiosensibilité d'un patient en fonction du nombre de foci pH2AX, pATM et MRE11 à l'état spontané (t0) et/ou après 10 min (t1), 1h (t2), 4h (t3) et 24h (t4)) de réparation post-irradiation sous 2Gy, du nombre de micronoyaux $N_{MN}(t)$ observés à l'état spontané et après 24h de temps de réparation après irradiation avec une dose absorbée de 2 Gy pour 100 cellules (en %), du nombre de petit foci pH2AX par cellule à l'état spontané et du pourcentage de gros noyaux (supérieurs à 150 $\mu m^2$) à t0 et ce, pour différents échantillons cellulaires de peau de patients

| lignée cellulaire | Nbre de foci pH2AX spontané (à $t_0$) | Nbre de foci pH2AX par cellule après 24h de réparation (à $t_4$) | Nbre moyen de micronoyaux radioinduits après 24h de réparation (à $t_4$) (%) | % de gros noyaux (%) | nbre de petit foci pH2AX à t0 | $N_{MRE11}$ (à $t_0$) | Nbre moyen de micronoyaux (à $t_0$) | $N_{pATM}$ après 10 min de réparation (à $t_1$) | $N_{pATM}$ après 1h de réparation (à $t_2$) | radiosensibilité de l'échantillon déterminée selon l'invention | grade de sévérité CTCAE déterminée selon l'invention |
|---|---|---|---|---|---|---|---|---|---|---|---|
| HF19 | 0±0 | 0,1±0,0 | 1±1 | 0 | 0 | 0±0 | 0±0 | 40±2 | 20±1 | groupe I | 1 |
| 19HM | 0.65±0.07 | 2.07±0.16 | 4.67±1.15 | 0 | 0 | 0±0 | 4.67±1.15 | 26±4 | 16.7±8.8 | groupe II | 2 |
| 29CLB | 1.45±0.23 | 1.9±0.34 | 10.67±1.15 | 0 | 43.33 | 0.3±0.3 | 4.33±0.58 | 20±6 | 23.3±3.3 | groupe II | 3 |
| 01DAX | 1.38±0.24 | 4.66±0.79 | 10.17±6.37 | 0 | 0 | 0±0 | 10.17±6.37 | 16.67±6.67 | 23.3±8.8 | groupe II | 3 |
| 13CLB | 0.69±0.1 | 1.25±0.21 | 8.33±3.11 | 0 | 0 | 0±0 | 3±1 | 13.33±3.33 | 15±3 | groupe II | 4 |
| 35CLB | 1.09±0.23 | 2.01±0.15 | 9.67±2 | 33 | 0 | 0±0 | 2±0 | 30±6 | 16.7±3.3 | groupe II | 2 |
| 01PAU | 2.45±1.21 | 1.65+0.13 | 5.33±1.15 | 1.67 | 43.33 | 0±0 | 5.33±1.15 | 0±0 | 23.3±4.4 | groupe II | 3 |
| AT2EM | 4.00±1.00 | 16.00±4.00 | 25.00±3.00 | 5 | 100 | 0±0 | 25.0±3.0 | 0±0 | 1±0 | groupe III | 5 |
| AT5BI | 3.00±1.00 | 17.00±6.00 | 30.00±3.00 | 5 | 100 | 0±0 | 30±3 | 0±0 | 0±0 | groupe III | 5 |
| AT4BI | 2.00±1.00 | 22.00±4.00 | 39.00±3.00 | 5 | 100 | 0±0 | 4±1 | 0±0 | 3±1 | groupe III | 5 |
| AT1BR | 2.00±1.00 | 12.00±4.00 | 28.00±3.00 | 5 | 100 | 0±0 | 4.2±1.0 | 0±0 | 1±0 | groupe III | 5 |
| 180BR | 3.00±1.00 | 35.00±3.00 | 37.00±3.00 | 5 | 100 | 0±0 | 4.5±2.0 | 36±2 | 20±1 | groupe III | - |

2.3 Evaluation prédictive du grade de sévérité et de la radiosensibilité de l'échantillon

Les cas de décès post-irradiation (grade de sévérité 5 selon la classification CTCAE) ont été décrits dans la littérature depuis les années 1970 jusqu'à nos jours et correspondent systématiquement, soit à des cas d'ataxie telangiectasique, soit au cas d'un patient atteint par une mutation de la ligase 4 (voir l'article de A. Joubert et al, « DNA double-strand break repair defects in syndromes associated with acute radiation response : At least two different assays to predict intrinsic radiosensitivity ? », paru dans International Journal of Radiation Biology, vol. 84(2), p107-125 (2008)). Sur la base de ces données rétrospectives et connaissant la dose totale cumulée lors de ces sessions de radiothérapie, le parallèle avec le nombre de cassures double-brin (CDB) non réparées correspondant a pu être fait. En effet, le nombre de cassures double-brin non réparées a été mesuré chez un grand nombre de lignées ataxiques et du cas unique de mutation de LIG4 (lignée cellulaire 180BR). Ces lignées montrent systématiquement un taux de cassures non réparées dépassant le seuil de létalité après une dose. Les valeurs des lignées cellulaires de patients AT et 180BR présentées dans le tableau 2 contiennent les preuves de la détermination du seuil à partir duquel le nombre de cassures non réparées est létal pour le patient. Pour ces cas particuliers, le grade CTCAE correspondant est 5 (=décès). Les grades de sévérité de 2 à 4 concernent des réactions tissulaires (ex : dermites, rectites, etc). Le grade de sévérité 1 concerne des effets secondaires supportables qui sont souvent confondus, suivant les praticiens avec le grade 0 (absence d'effet). La figure 6 (A) représente le nombre maximum de foci obtenus avec le marqueur pH2AX après 24h de réparation post-irradiation en fonction du nombre maximum de foci pATM de différents patients, montre la corrélation existant entre le grade de sévérité selon la classification CTCAE et ces paramètres.

Ainsi, pour différents échantillons cellulaires de peau de patients (cf. tableau 2), le grade de sévérité selon la classification CTCAE a été déterminé via les formules précédemment décrites, à savoir :

$$CTCAE = 5 - Max[N_{pATM}(t1) ; N_{pATM}(t2)]/10.$$

Cette formule est particulièrement adaptée à la détermination du grade de sévérité de patients appartenant au groupe de radiosensibilité de type II (radiosensibilité modérée). Concernant les patients appartenant au groupe de radiosensibilité de type III (patients radiosensibles), tous ces patients présentent et doivent présenter un grade de sévérité 5. Pour ces patients radiosensibles, la radiothérapie est prohibée.

En pratique, le groupe de radiosensibilité du patient est déterminée avant celle du grade de sévérité selon la classification de la CTCAE, et ce, afin d'attribuer au patient une valeur correcte de grade de sévérité. A ce jour, seul un cas de lignée cellulaire n'a pas validée la formule de détermination du grade de sévérité selon la formule précédente. C'est le cas de la lignée cellulaire (180BR) appartenant à un groupe de radiosensibilité de type III, pour laquelle on calcule un grade de sévérité selon la classification CTCAE basée sur la formule précédente d'une valeur de 1. Toutefois, l'utilisation de la formule prédisant le grade de sévérité doit avantageusement s'effectuer après avoir déterminé le groupe de radiosensibilité (groupe I, II ou III) du patient. Ainsi, le patient 180BR appartient bien selon l'invention au groupe III (hyper-radiosensibilité) et donc, à ce titre ne doit pas être soumis aux radiations, et ce, sous aucun prétexte. En revanche, le grade de sévérité des patients a été validé pour toutes les autres lignées cellulaires.

Le groupe de radiosensibilité de l'échantillon a été déterminée via les formules précédemment décrites, de la manière suivante :

(a) on considère que l'échantillon est radiorésistant si $N_{pH2AX}(t4) < 2$ et $N_{pATM}(t1) > N_{pATM}(t2)$ et $N_{pATM}(t1) > 30$ et $A < 10$ et $B < 5$ et $C < 2$ ; sachant que :

$$C = N_{pH2AX}(t0) + N_{MN}(t0) ;$$

$$B = \% \text{ de gros noyaux (supérieurs à 150 µm2) à t0 ;}$$

$$A = N_{MRE11}(t0) + \text{Nbre de petit foci pH2AX/cellule à t0 ;}$$

(b) on considère que l'échantillon est très radiosensible si ($N_{pH2AX}(t4) > 8$ ou $N_{MN}(t4) > 24$);
(c) on considère que l'échantillon présente une radiosensibilité modérée pour toutes les autres conditions.

Les valeurs quantitatives du grade de sévérité selon la classification CTCAE et de la radiosensibilité de l'échantillon sont indiquées dans le tableau 2.

**Revendications**

1. Procédé pour caractériser la radiosensibilité d'un échantillon cellulaire envers un rayonnement ionisant, ledit échantillon cellulaire ayant été obtenu à partir de cellules prélevées sur un patient dans une zone non irradiée ou peu irradiée, dans lequel procédé :
on amplifie lesdites cellules prelévées, ces cellules amplifiées constituant « l'échantillon cellulaire » ;

(i) on détermine sur ledit échantillon cellulaire le nombre moyen de foci nucléaires obtenus avec au moins deux des marqueurs pH2AX, pATM et MRE11 aux temps d'observation t (ces nombres moyens étant appelé respectivement $N_{pH2AX}(t)$, $N_{pATM}(t)$, $N_{MRE11}(t)$), lesdits temps d'observation t étant le temps t=0 min (appelé t0, état non irradié) et au moins un temps d'observation sélectionné parmi t = t1, t2 et t4 après irradiation avec une dose absorbée D;
(ii) on détermine au moins un paramètres sélectionné dans le groupe formé par:

- le grade de sévérité de la réaction tissulaire post-radiothérapie selon la classification CTCAE, exprimé comme paramètre sans dimension, comme CTCAE = 5 - Max[$N_{pATM}$ (t1) ; $N_{pATM}$ (t2)]/10;
- le groupe de radiosensibilité de l'échantillon, en utilisant au moins les nombres moyens $N_{pH2AX}(t)$, $N_{pATM}(t)$ et $N_{MRE11}(t)$ de la manière suivante :

  • Si $N_{pH2AX}(t4) < 2$ et

    si $N_{pATM}(t1) > N_{pATM}(t2)$ et
    si $N_{pATM}(t1) > 30$ et
    si A < 10 et
    si B < 5 et
    si C < 2

  où

$$C = N_{pH2AX}(t0) + N_{MN}(t0) ;$$

  où

$$B = \% \text{ de gros noyaux (supérieurs à 150 } \mu m^2 \text{) à t0 ;}$$

  où

$$A = N_{MRE11} (t0) + \text{Nbre de petit foci pH2AX/cellule à t0 ;}$$

alors le groupe de radiosensibilité (critère GROUPE) est considéré comme « Groupe I » (cellules radiorésistantes) ;
• Si ($N_{pH2AX}(t4) > 8$ ou $N_{MN}(t4) > 24$)
alors le critère GROUPE est considère comme « Groupe III » (cellules très radiosensibles),
• pour toutes les autres conditions, on considère que le critère GROUPE est « Groupe II » (cellules montrant une radiosensibilité modérée);

dans lequel $N_{MN}(t)$ est le nombre moyen de micronoyaux observés au temps t pour 100 cellules (en %) et dans lequel procédé

• t4 est une valeur fixe qui représente le temps pour lequel le taux de cassures ADN atteint sa valeur résiduelle, et de préférence comprise entre 12h et 48h, et qui est encore plus préférentiellement d'environ 24 heures ;
• t2 est une valeur fixe qui représente le temps au bout duquel environ 50% des CDB sont réparées dans des cellules témoins issues de patients radiorésistants, et qui est avantageusement choisie entre 5 fois t1 et 7 fois t1, mais qui doit dans ce cas être au moins de 35 minutes et au plus de 90 minutes, et est de préférence compris entre 45 minutes et 75 minutes, et est encore plus préférentiellement environ 60 minutes ;

• t1 est une valeur fixe qui représente le temps au bout duquel le nombre de CDB reconnues atteint son maximum dans des cellules témoins issues de patients radiorésistants, et qui est avantageusement chosie entre 5 minutes et 15 minutes après l'arrêt de l'irradiation, de préférence entre 7,5 minutes et 12,5 minutes, et encore plus préferentiellement à environ 10 minutes.

2. Procédé selon la revendication 1, dans lequel on détermine en plus sur ledit échantillon cellulaire le nombre moyen de micronoyaux observés aux temps t pour 100 cellules [en %] (ce nombre moyen étant appelé $N_{MN}(t)$), les temps t étant au moins t0 (non irradié) et t4 après irradiation avec une dose absorbée D.

3. Procédé selon la revendication 1 ou 2, dans lequel on détermine les nombres moyens $N_{pH2AX}(t)$ à t= t0, t1, t2 et t4 et les nombres $N_{pATM}(t)$ à t=t0, t1 et t2, et les nombres $N_{MRE11}(t)$ à t= t0, t1 et t2.

4. Procédé selon l'une quelconque des revendications 1 à 3, dans lequel lesdites cellules prélevées sont des cellules fibroblastiques issues d'une biopsie de peau d'un patient.

5. Procédé selon l'une quelconque des revendication 1 à 4, dans lequel la dose absorbée D est comprise entre 0,5 Gy et 4 Gy, de préférence comprise entre 1 Gy et 3 Gy, de préférence entre 1,7 Gy et 2,3 Gy, et est encore plus préférentiellement de 2 Gy.

6. Procédé selon l'une quelconque des revendications 1 à 5, dans lequel t1 est compris entre 8 minutes et 12 minutes, t2 est compris entre 50 minutes et 70 minutes, et t4 est compris entre 22 heures et 26 heures.

7. Procédé selon la revendication 5 ou 6, dans lequel t1 est 10 minutes, t2 est 60 minutes, t4 est 24 heures, et D est 2 Gy.

8. Procédé selon l'une quelconque des revendications 2 à 7, dans lequel on détermine la radiosensibilité de la manière suivante :

(a) on considère que l'échantillon est radiorésistant si $N_{pH2AX}(t4) < 2$ et $N_{pATM}(t1) > N_{pATM}(t2)$ et $N_{pATM}(t1) > 30$ et A< 10 et B < 5 et C < 2 ; sachant que :

$$C = N_{pH2AX}(t0) + N_{MN}(t0) ;$$

$$B = \% \text{ de gros noyaux (supérieurs à 150 µm}^2\text{) à t0 ;}$$

$$A = N_{MRE11}(t0) + \text{Nbre de petit foci pH2AX/cellule à t0 ;}$$

(b) on considère que l'échantillon est très radiosensible si ($N_{pH2AX}(t4) > 8$ ou $N_{MN}(t4) > 24$) ;
(c) on considère que l'échantillon présente une radiosensibilité modérée pour toutes les autres conditions.

9. Procédé selon l'une quelconque des revendications 1 à 8, dans lequel la détermination de $N_{pH2AX}$, $N_{pATM}$ et/ou $N_{MRE11}$ fait intervenir un test d'immunofluorescence.

10. Procédé selon l'une quelconque des revendications 1 à 9, dans lequel lesdites cellules témoins issues de patients radiorésistants ont été sélectionnées en tant que cellules montrant un taux de survie clonogénique *in vitro* supérieur à 55% après une irradiation avec une dose absorbée de 2 Gy.

11. Procédé selon l'une quelconque des revendications 1 à 10, dans lequel lesdites cellules témoins issues de patients radiorésistants ont été sélectionnées en tant que cellules prélevées sur des patients n'ayant pas montré de réactions tissulaires significatives pendant ou à la suite d'un traitement radiothérapeutique.

**Patentansprüche**

1. Verfahren zur Charakterisierung der Strahlenempfindlichkeit einer Zellprobe gegenüber ionisierender Strahlung, wobei die Zellprobe aus Zellen gewonnen wurde, die einem Patienten in einem nicht bestrahlten oder wenig bestrahlten Bereich entnommen wurden, wobei in diesem Verfahren:
die Zellen amplifiziert werden und diese amplifizierten Zellen die "zelluläre Probe" darstellen;

i) an der Zellprobe die mittlere Anzahl von nukleären Foci bestimmt wird, die mit mindestens zwei der Marker pH2AX, pATM und MRE11 zu den Beobachtungszeiten t erhalten wurden (diese mittleren Anzahlen werden jeweils als $N_{PH2Ax}(t)$, $N_{pATM}(t)$, $N_{MRE11}(t)$ bezeichnet), wobei diese Beobachtungszeiten t die Zeit t = 0 min (als t0 bezeichnet, unbestrahlter Zustand) ist und mindestens eine Beobachtungszeit ausgewählt aus t = t1, t2 und t4 nach Bestrahlung mit einer absorbierten Dosis D;

(ii) mindestens ein Parameter bestimmt wird, ausgewählt aus der Gruppe bestehend aus:

- dem Schweregrad der Gewebereaktion nach der Strahlentherapie gemäß der CTCAE-Klassifikation, ausgedrückt als dimensionsloser Parameter gemäss

$$CTCAE = 5 - Max [N_{pATM}(t1); N_{pATM}(t2)] / 10;$$

- der Gruppe der Strahlenempfindlichkeit der Zellprobe, unter Verwendung von mindestens einer der mittleren Anzahlen $N_{PH2Ax}(t)$, $N_{pATM}(t)$ und $N_{MRE11}(t)$, und zwar folgendermassen:

-- wenn $N_{pH2Ax}(t4) < 2$ und
wenn $N_{pATM}(t1) > N_{PATM}(t2)$ und
wenn $N_{pATM}(t1) > 30$ und
wenn A < 10 und
wenn B < 5 und
wenn C < 2
wobei

$$C = N_{pH2Ax}(t0) + N_{MN}(t0);$$

wobei

$$B = \% \text{ der großen Kerne (größer als 150 μm}^2) \text{ bei } t0;$$

wobei

$$\text{wobei } A = N_{MRE11}(t0) + \text{Anzahl der kleinen Foci pH2AX / Zelle bei t0;}$$

dann wird die Gruppe der Strahlenempfindlichkeit (Kriterium GRUPPE) als Gruppe I bezeichnet (strahlenresistente Zellen);
-- wenn ($N_{pH2Ax}(t4) > 8$ oder $N_{MN}(t4) > 24$), dann wird das Kriterium GRUPPE als "Gruppe III" (hoch strahlenempfindliche Zellen) angesehen;
-- für alle anderen Bedingungen wird das Kriterium GRUPPE als "Gruppe II" (Zellen mit mäßiger Strahlenempfindlichkeit) eingestuft;

wobei $N_{MN}(t)$ die mittlere Anzahl (in % ausgedrückt) von Mikrokernen, zur Zeit t für 100 Zellen beobachtet, bedeutet; und wobei in diesem Verfahren

- t4 ein fester Wert ist, der die Zeit darstellt, für die die Rate der DNA-Brüche ihren Restwert erreicht, und der vorzugsweise zwischen 12 und 48 Stunden liegt und noch bevorzugter etwa 24 Stunden beträgt;
- t2 ein fester Wert ist, der die Zeit darstellt, nach der etwa 50% der DSBs in Kontrollzellen von strahlenresistenten Patienten repariert werden, und der vorteilhafterweise zwischen dem 5-fachen von t1 und dem 7-fachen von t1 gewählt wird, in diesem Fall aber mindestens 35 Minuten und höchstens 90 Minuten betragen muss, und der vorzugsweise zwischen 45 Minuten und 75 Minuten liegt, und noch bevorzugter bei etwa 60 Minuten liegt; - t1 ein fester Wert ist, der die Zeit darstellt, nach der die Anzahl der erkannten DSBs in Kontrollzellen von strahlenresistenten Patienten ihr Maximum erreicht, und der vorteilhafterweise zwischen 5 Minuten und 15 Minuten nach Beendigung der Bestrahlung ausgewählt wird, bevorzugt zwischen 7,5 Minuten und 12,5 Minuten liegt, und noch bevorzugter bei ungefähr 10 Minuten liegt.

2. Verfahren nach Anspruch 1, wobei man zusätzlich an der Zellprobe die mittlere Anzahl der zu Zeitpunkten t beobachteten Mikrokerne pro 100 Zellen [in %] bestimmt (diese durchschnittliche Anzahl wird als $N_{MN}(t)$ bezeichnet), wobei die Zeitpunkte t mindestens sind t0 (nicht bestrahlt) und t4 nach Bestrahlung mit einer Energiedosis D.

3. Verfahren nach Anspruch 1 oder 2, wobei die Durchschnittszahlen $N_{pH2Ax}(t)$ bei t = t0, t1, t2 und t4 und die Anzahlen $N_{pATM}(t)$ bei t = t0, t1 und t2, und die Anzahlen $N_{MRE11}(t)$ bei t = t0, t1 und t2 bestimmt werden.

4. Verfahren nach einem der Ansprüche 1 bis 3, wobei die entnommenen Zellen Fibroblastenzellen sind, die aus einer Hautbiopsie eines Patienten stammen.

5. Verfahren nach einem der Ansprüche 1 bis 4, bei dem die absorbierte Dosis D zwischen 0,5 Gy und 4 Gy, vorzugsweise zwischen 1 Gy und 3 Gy, vorzugsweise zwischen 1,7 Gy und 2,3 Gy liegt, und noch bevorzugter 2 Gy beträgt.

6. Verfahren nach einem der Ansprüche 1 bis 5, wobei t1 zwischen 8 Minuten und 12 Minuten liegt, t2 zwischen 50 Minuten und 70 Minuten, und t4 zwischen 22 Stunden und 26 Stunden.

7. Verfahren nach Anspruch 5 oder 6, wobei t1 10 Minuten beträgt, t2 60 Minuten beträgt, t3 4 Stunden beträgt, t4 24 Stunden beträgt und D 2 Gy beträgt.

8. Verfahren nach einem der Ansprüche 2 bis 7, wobei die Strahlenempfindlichkeit auf folgende Weise bestimmt wird:

(a) Die Probe wird als strahlenresistent angesehen, wenn $N_{pH2Ax}(t4) < 2$ und $N_{pATM}(t1) > N_{pATM}(t2)$ und $N_{pATM}(t1) > 30$ und $A < 10$ und $B < 5$ und $C < 2$; wobei:

$$C = N_{pH2Ax}(t0) + N_{MN}(t0);$$

$$B = \% \text{ der großen Kerne (größer als } 150 \text{ μm}^2) \text{ bei } t0;$$

$$A = N_{MRE11}(t0) + \text{Anzahl der kleinen Foci } p_{H2AX} / \text{Zelle bei } t0;$$

(b) die Probe wird als hoch strahlenempfindlich angesehen, wenn ($N_{pH2Ax}(t4) > 8$ oder $N_{MN}(t4) > 24$);
(c) die Probe wird für alle anderen Bedingungen als mäßig strahlenempfindlich eingestuft.

9. Verfahren nach einem der Ansprüche 1 bis 8, wobei die Bestimmung von $N_{pH2AX}$, $N_{pATM}$ und / oder $N_{MRE11}$ einen Immunfluoreszenztest beinhaltet.

10. Verfahren nach einem der Ansprüche 1 bis 10, wobei die Kontrollzellen von strahlenresistenten Patienten als Zellen ausgewählt wurden, die nach Bestrahlung mit einer absorbierten Dosis von 2 Gy eine klonogene Invitro-Überlebensrate von mehr als 55% aufweisen.

11. Verfahren nach einem der Ansprüche 1 bis 10, wobei die Kontrollzellen von strahlenresistenten Patienten als Zellen ausgewählt wurden, die Patienten entnommen wurden, die während oder nach einer strahlentherapeutische Behandlung keine signifikanten Gewebereaktionen zeigten.

**Claims**

1. Method for characterizing the radiosensitivity to ionizing radiation of a cellular sample, said cellular sample having being obtained from cells taken from a patient in a non-irradiated or only slightly irradiated zone, in which method: said sampled cells are amplified, said amplified cells constituting the "cellular sample";

   (i) determining on said cellular sample the mean number of nuclear foci obtained having at least two of the markers pH2AX, pATM and MRE11 at the time of observation t (said mean numbers being called respectively $N_{pH2AX}(t)$, $N_{pATM}(t)$, $N_{MRE11}(t)$), said observation time t being the time t = 0 min (referred to as t0, the non-irradiated state) and at least one observation time selected from t = t1, t2, t3 and t4 after irradiation with an absorbed dose D;
   (ii) determining at least one parameter selected from the group consisting of:

   - the severity grade of the post-radiotherapy tissular reaction according to the CTCAE classification, expressed as a dimensionless parameter, as

$$CTCAE = 5 - Max[N_{pATM}(t1); N_{pATM}(t2)]/10 ;$$

   - the radiosensitivity group score of the sample, by using at least the mean numbers $N_{pH2Ax}(t)$, $N_{pATM}(t)$ and $N_{MRE11}(t)$ in the following manner:

     -- if $N_{pH2AX}(t4) < 2$ and
     if $N_{pATM}(t1) > N_{pATM}(t2)$ and
     if $N_{pATM}(t1) > 30$ and
     if $A < 10$ and
     if $B < 5$ and
     if $C < 2$
     where

$$C = N_{pH2AX}(t0) + N_{MN}(t0);$$

     where

$$B = \% \text{ of large nuclei (greater than 150 } \mu m^2) \text{ at } t0;$$

     where

$$A = N_{MRE11}(t0) + \text{Number of small pH2AX foci/cell at } t0;$$

     then the radiosensitivity group (GROUP criterion) is considered to be "Group I" (radioresistant cells);
     -- if ($N_{pH2AX}(t4) > 8$ or $N_{MN}(t4) > 24$
     then the GROUP criterion is considered to be "Group III" (highly radiosensitive cells).
     -- for all other conditions, it is considered that the GROUP criterion is "Group II" (cells exhibiting moderate radiosensitivity);
     wherein $N_{MN}(t)$ is the mean number of micronuclei observed at time t for 100 cells [in %];
     and in which method
     -- t4 is a fixed value representing the time taken for the rate of DNA breaks to attain the residual value thereof, preferably between 12 h and 48 h, and which is more preferably approximately 24 hours;
     -- t2 is a fixed value representing the time at the end of which approximately 50% of the DSB are repaired in the control cells taken from radioresistant patients, and which is advantageously chosen to be between 5 times t1 and 7 times t1, but in this case must be at least 35 minutes and at most 90 minutes, and is preferably between 45 minutes and 75 minutes, and is more preferably approximately 60 minutes;

-- t1 is a fixed value representing the time at the end of which the number of DSB recognized has reached a maximum in the control cells taken from radioresistant patients, and which is advantageously chosen to be between 5 minutes and 15 minutes after the end of irradiation, preferably between 7.5 minutes and 12.5 minutes, and yet more preferably to be around 10 minutes.

2. Method according to claim 1, in which, on the said cellular sample, the mean number of micronuclei observed is also determined at time t for 100 cells [in %] (this number being referred to as $N_{MN}(t)$), the time t being at least t0 (non-irradiated) and t4 after irradiation with an absorbed dose D.

3. Method according to claims 1 or 2, in which the mean numbers $N_{pH2AX}(t)$ at t = t0, t1, t2 and t4 and the numbers $N_{pATM}(t)$ at t = t0, t1 and t2, and the numbers $N_{MRE11}(t)$ at t = t0, t1, and t2 are determined.

4. Method according to any of claims 1 to 3, in which said sampled cells are fibroblast cells taken from a skin biopsy of a patient.

5. Method according to any of claims 1 to 4, in which the absorbed dose D is between 0.5 Gy and 4 Gy, preferably between 1 Gy and 3 Gy, more preferably between 1.7 Gy and 2.3 Gy, and still more preferably 2 Gy.

6. Method according to any of claims 1 to 5, in which t1 is taken to be between 8 minutes and 12 minutes, t2 is taken to be between 50 minutes and 70 minutes, and t4 is taken to be between 22 hours and 26 hours.

7. Method according to claims 5 or 6, in which t1 is 10 minutes, t2 is 60 minutes, t4 is 24 hours, and D is 2 Gy.

8. Method according to any of claims 2 to 7, in which the radiosensitivity is determined in the following manner:

(a) the sample is considered to be radioresistant if $N_{pH2Ax}(t4) < 2$ and $N_{pATM}(t1) > N_{pATM}(t2)$ and $N_{pATM}(t1) > 30$ and A < 10 and B < 5 and C < 2; given that:

$$C = N_{pH2AX}(t0) + N_{MN}(t0);$$

$$B = \text{\% of large nuclei (greater than 150 } \mu m^2) \text{ at t0;}$$

$$A = N_{MRE11}(t0) + \text{Number of small pH2AX foci/cell at t0;}$$

(b) the sample is considered to be highly radiosensitive if $(N_{pH2AX}(t4) > 8$ or $N_{MN}(t4) > 24$;
(c) the sample is considered to have a moderate radiosensitivity under all other conditions.

9. Method according to any of claims 1 to 8, in which determination of $N_{pH2AX}$, $N_{pATM}$ and/or $N_{MRE11}$ involves an immunofluorescence test.

10. Method according to any of claims 1 to 9, in which said control cells taken from radioresistant patients have been selected as cells demonstrating an *in vitro* clonogenic survival rate greater than 55% after irradiation with an absorbed dose of 2 Gy.

11. Method according to any of claims 1 to 10, in which said control cells from radioresistant patients have been selected as cells taken from patients who have not demonstrated significant tissular reactions during or following a radiotherapy treatment.

Figure 1

**Figure 2**

Figure 3

Figure 4

Figure 5

Figure 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2004013634 A
- US 20070072210 A
- EP 2466310 A1
- WO 0047760 A
- US 7279290 B
- EP 1616011 B1
- WO 0190408 A
- WO 2004059004 A
- WO 2006136686 A
- KR 20030033519
- KR 20030033518

- US 2011312514 A
- US 20070264648 A
- US 2008234946 A
- US 2012041908 A
- WO 2014154854 A
- WO 2013187973 A
- US 8269163 B
- WO 201088650 A
- WO 2010136942 A
- WO 2010109357 A

**Littérature non-brevet citée dans la description**

- **N. FORAY et al.** A subset of ATM- and ATR-dependent phosphorylation events requires the BRCA1 protein. *EMBO Journal,* 2003, vol. 22 (11), 2860-2871
- **JOUBERT et al.** DNA double-strand break repair defects in syndromes associated with acute radination response ; At least two different assays to predict intrinsic radiosensitivity ?. *Int. J. Radiat. Biol.,* 2008, vol. 84 (2), 107-125
- **N. FORAY.** Les réparatoses : nouveaux concepts sur la prédiction de la radiosensibilité. *Rencontres Nucléaire & Santé,* 25 Janvier 2008

- *CHEMICAL ABSTRACTS,* 28718-90-3
- **ELKIN M. et al.** The radiobiology of cultured mammalian cells. Gordon and Breach, 1967
- *CHEMICAL ABSTRACTS,* 9002-93-1
- **A. JOUBERT et al.** DNA double-strand break repair defects in syndromes associated with acute radiation response : At least two different assays to predict intrinsic radiosensitivity ?. *International Journal of Radiation Biology,* 2008, vol. 84 (2), 107-125